# EUROPEAN PATENT APPLICATION

(11) **EP 3 932 920 A1**
(43) Date of publication of application: **05.01.2022**
(21) Application number: 20765627.3
(22) Date of filing: 02.03.2020
(51) Int. Cl.: C07D 471/04

(54) **METHOD FOR PREPARING TRICYCLIC COMPOUND, AND INTERMEDIATE THEREOF**

(30) Priority: 01.03.2019 CN 201910155700; 13.03.2019 CN 201910187667
(71) Applicant: KBP Biosciences Co., Ltd., Jinan, Shandong 250101 (CN)
(72) Inventor: HUANG, Zhenhua, Jinan, Shandong 250101 (CN); GUO, Pengfei, Jinan, Shandong 250101 (CN); LI, Cheng, Jinan, Shandong 250101 (CN)
(74) Representative: Patentanwälte Magenbauer & Kollegen Partnerschaft mbB
(86) International application number: PCT/CN2020/077413
(87) International publication number: WO 2020/177658

(57) **Abstract**

The present application discloses a process for preparing a fused tricyclic compound and an intermediate thereof, specifically relates to a process for preparing a (3 S,3 aR)-3 -cyclopentyl-3,3 a,4,5 -tetrahydro-2H-pyrazolo [3,4-f]quinoline compound, intermediates in said process, and methods for preparing said intermediates.

## Description

### Technical Field

The present invention discloses a process for preparing a fused tricyclic compound and an intermediate thereof, and specifically relates to a process for preparing a (3 S,3 aR)-3 -cyclopentyl-3,3 a,4,5 -tetrahydro-2H-pyrazolo [3,4-f]quinoline compound, particularly relates to a process for preparing (3 S,3 aR)-2-(3 -chloro-4-cyanophenyl)-3 -cyclopentyl-3,3a,4,5 -tetrahydro-2H-pyrazolo [3,4-f]quinol ine-7-carboxylic acid or 2-chloro-4-[(3S,3aR)-3-cyclopentyl-7-(4-hydroxylpiperidine-1-carbonyl)-3,3a,4,5-tetrahydro-2H-pyrazolo[3,4-f]quinoline-2-yl]benzonitrile, and discloses intermediates useful in the synthesis of said compound.

### Background

The process for preparing a (3S,3aR)-3-cyclopentyl-3,3a,4,5-tetrahydro-2H-pyrazolo[3,4-f]quinoline compound (for example (3S,3aR)-2-(3-chloro-4-cyanophenyl)-3-cyclopentyl-3,3a,4,5-tetrahydro-2H-pyrazolo[3,4-f]quinol ine-7-carboxylic acid (a compound of formula II), 2-chloro-4- [(3 S,3 aR)-3-cyclopentyl-7-(4-hydroxylpiperidine-1-carbonyl)-3,3a,4,5 -tetrahydro-2H-pyrazolo[3,4-f]quinoline-2-yl]benzonitrile (a compound of formula I)) is disclosed in WO2012022121A1 or WO2014094664A1. The resolution steps are all performed by supercritical liquid chromatography (SFC), and the preparation processes have low product yield and high production cost.

In addition, the reactant m-chloroperoxybenzoic acid used in the process reaction in WO2014094664A1 has high risk, and trimethylsilyl cyanide is extremely toxic, so the process of the (3S,3aR)-3-cyclopentyl-3,3a,4,5-tetrahydro-2H-pyrazolo[3,4-f]quinoline compound needs to be optimized to meet the requirement of large-scale industrial production.

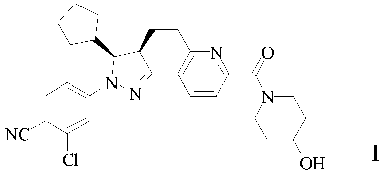

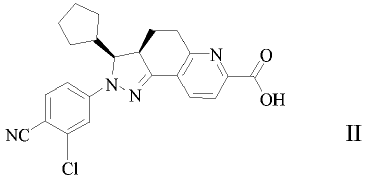

### Summary of the Invention

An object of the present invention is to provide a process for preparing the (3S,3aR)-3-cyclopentyl-3,3a,4,5-tetrahydro-2H-pyrazolo[3,4-f]quinoline compound, in which a chiral base is used to replace the supercritical liquid chromatography (SFC) for chiral resolution, the production scale is increased to a batch size suitable for the commercial-scale preparation, and the purpose of large-scale industrial production is realized. The total reaction yield is improved compared with the published process, the total production rate is improved by about 7 times, and the process of the present invention is safer.

In order to achieve the above object, the present invention contemplates a series of chiral purification methods, such as enzymatic hydrolysis, chiral acid/base resolution, chiral group induction, simulated moving bed chromatography (SMB) resolution, crystal separation of asymmetric derivatives, silica gel column separation of asymmetric derivatives. Among others, the chiral base resolution has a better resolution effect, and other resolution methods do not achieve the resolution object. As for the chiral base resolution method, several dozens of chiral base reagents have been tried, among which (1S,2S)-cyclohexane diamine, (1R,2R)-cyclohexane diamine, quinine, quinidine, and deuterated derivatives thereof have good resolution effect. For the chiral base(s) with the remarkable resolution effect, various experimental solvents are tried, among others, a ketone solvent, an ester solvent, or tetrahydrofuran has a better resolution effect. Said ketone solvent is selected from acetone, butanone, pentanone, methyl isobutyl ketone, and the like, preferably acetone. Said ester solvent is selected from ethyl acetate, isopropyl acetate, tert-butyl acetate, and the like, preferably ethyl acetate. For example, (1S,2S)-cyclohexane diamine or (1R,2R)-cyclohexane diamine has a relatively good resolution effect in acetone, and quinine or quinidine has a relatively good resolution effect in ethyl acetate.

The present invention aims to claim the following technical solutions:
Solution 1: A process for preparing a compound of formula (III), which comprises reacting a compound of formula (IV) and a chiral base Y to produce the compound of formula (III), said chiral base Y is selected from (1S,2S)-cyclohexane diamine, (1R,2R)-cyclohexane diamine, quinine, quinidine or deuterated derivatives thereof. 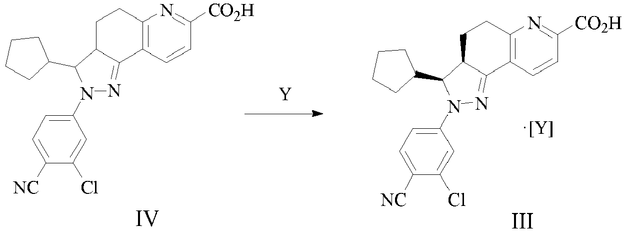
Solution 2: The process of Solution 1, which comprises a reaction in a ketone solvent, an ester solvent, or tetrahydrofuran.
   Specific examples of reactions for Solutions 1 and 2 can be referred to the following Aspects 1-12 of the present invention, particularly Aspect 1.
Solution 3: A process for preparing a compound of formula (VIII), which comprises the following steps: 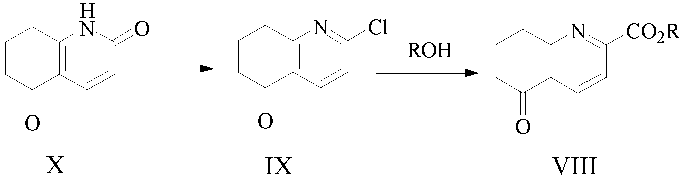
   (a) A compound of formula (IX) is prepared from a compound of formula (X), preferably the compound of formula (IX) is prepared from the compound of formula (X) and an acyl chloride compound, wherein the acyl chloride compound is selected from phosphoryl chloride, carbonyl chloride, and sulfuric chloride, for example, phosphorus oxychloride, acetyl chloride, and thionyl chloride;
   (b) The compound of formula (VIII) is prepared from the compound of formula (IX) and ROH, preferably the compound of formula (VIII) is prepared from the compound of formula (IX) and ROH in the presence of CO, wherein R is C₁₋₆alkyl.
   Specific examples of reactions for Solution 3 can be referred to the following Aspects 1-12 of the present invention, particularly Aspect 6.
Solution 4: The process of Solution 1, which comprises the following steps: 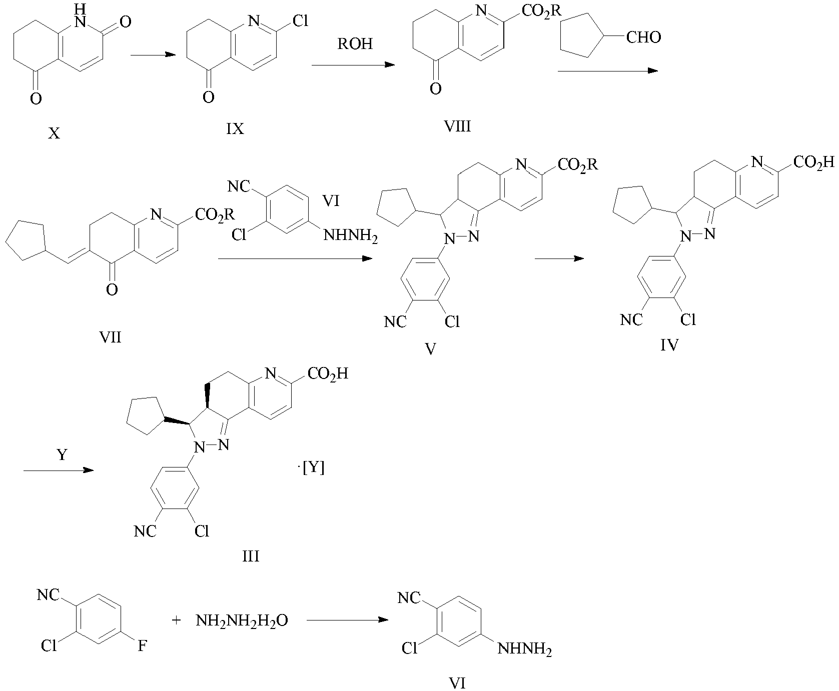
   (a) A compound of formula (IX) is prepared from a compound of formula (X), preferably the compound of formula (IX) is prepared from the compound of formula (X) and an acyl chloride compound, wherein the acyl chloride compound is selected from phosphoryl chloride, carbonyl chloride, and sulfuric chloride, for example, phosphorus oxychloride, acetyl chloride, and thionyl chloride;
   (b) A compound of formula (VIII) is prepared from the compound of formula (IX) and ROH, preferably the compound of formula (VIII) is prepared from the compound of formula (IX) and ROH in the presence of CO, wherein R is C₁₋₆alkyl;
   (c) The compound of formula (VIII) and cyclopentyl formaldehyde are reacted to produce a compound of formula (VII);
   (d) 2-chloro-4-fluoro benzonitrile and hydrazine hydrate are reacted to produce a compound of formula (VI);
   (e) The compound of formula (VII) and the compound of formula (VI) are reacted to produce a compound of formula (V);
   (f) The compound of formula (V) is hydrolyzed to produce the compound of formula (IV);
   (g) The compound of formula (IV) and a chiral base Y are reacted to produce the compound of formula (III).
   Specific examples of reactions for Solution 4 can be referred to the following Aspects 1-12 of the present invention, particularly Aspect 8.
Solution 5: A process for preparing a compound of formula (I), comprising the following steps: 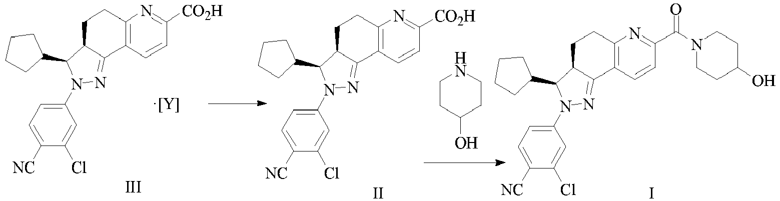
   (h) A compound of formula (III) is added to an acid solution and reacted to produce a compound of formula (II);
   (i) the compound of formula (II) and 4-hydroxylpiperidine are reacted to produce the compound of formula (I), wherein Y is selected from (1S,2S)-cyclohexane diamine, (1R,2R)-cyclohexane diamine, quinine, quinidine or deuterated derivatives thereof.
   Specific examples of reactions for Solution 5 can be referred to the following Aspects 1-12 of the present invention, particularly Aspects 2 and 3.
Solution 6: The process of Solution 5, comprising the following steps: 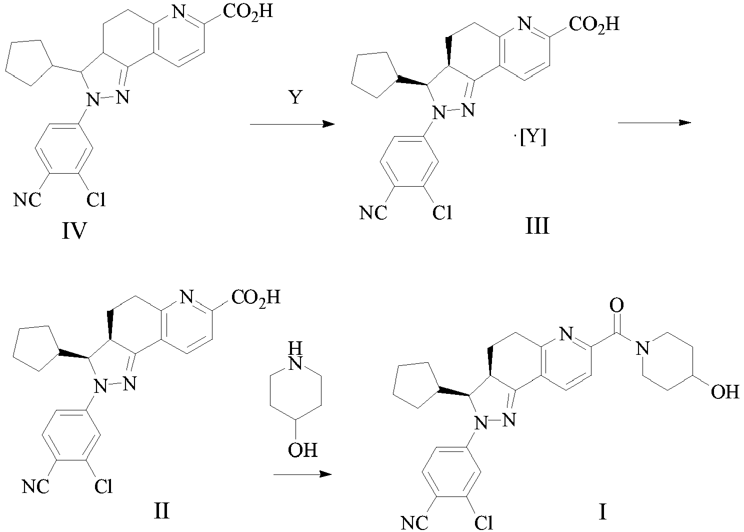
   (g) A compound of formula (IV) and a chiral base Y are reacted to produce the compound of formula (III);
   (h) The compound of formula (III) is added to an acid solution and reacted to produce the compound of formula (II);
   (i) the compound of formula (II) and 4-hydroxylpiperidine are reacted to produce the compound of formula (I).
   Specific examples of reactions for Solution 6 can be referred to the following Aspects 1-12 of the present invention, particularly Aspects 1, 2, and 3.
Solution 7: A process for preparing a compound of formula (I), comprising the following steps: 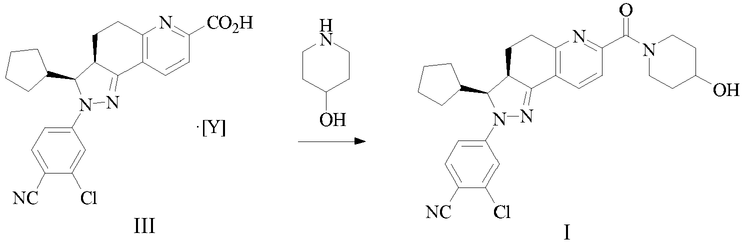
   Said process comprises: a compound of formula (III) and 4-hydroxylpiperidine are reacted to produce the compound of formula (I), wherein Y is selected from (1S,2S)-cyclohexane diamine, (1R,2R)-cyclohexane diamine, quinine, quinidine or deuterated derivatives thereof.
   Specific examples of reactions for Solution 7 can be referred to the following Aspects 1-12 of the present invention, particularly Aspect 4.
Solution 8: The compound of formula (III), having the following structure, 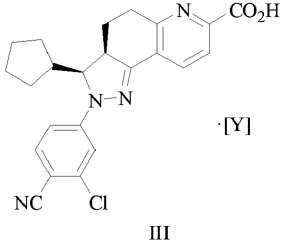 wherein Y is selected from (1S,2S)-cyclohexane diamine, (1R,2R)-cyclohexane diamine, quinine, quinidine, or deuterated derivatives thereof.
   Specific examples of reactions for Solution 8 can be referred to the following Aspects 1-12 of the present invention, particularly Aspect 5.
Solution 9: The process of Solution 6, comprising the following steps: 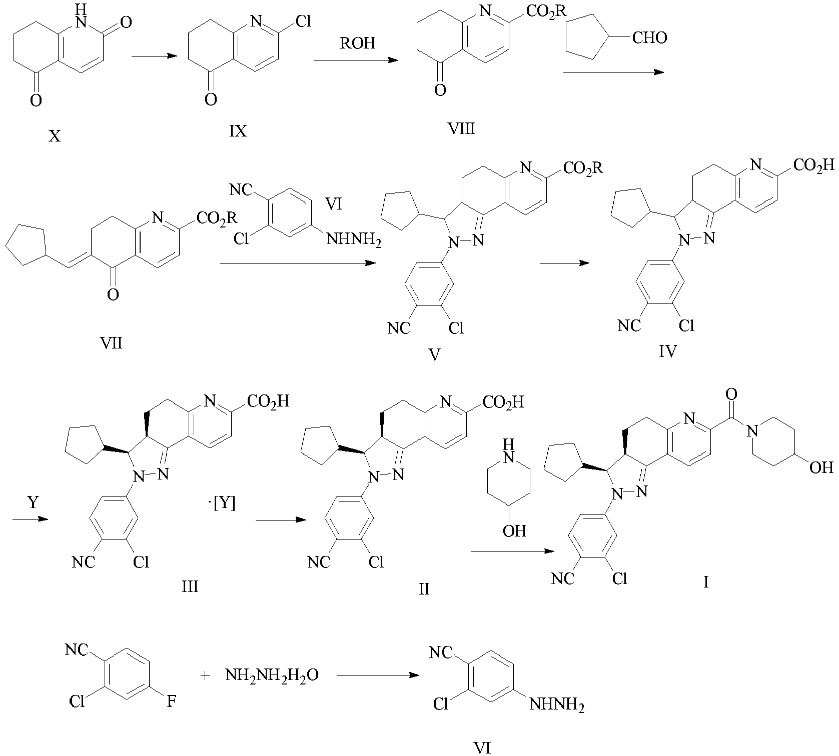
   (a) A compound of formula (IX) is prepared from a compound of formula (X), preferably the compound of formula (IX) is prepared from the compound of formula (X) and an acyl chloride compound, wherein the acyl chloride compound is selected from phosphoryl chloride, carbonyl chloride, and sulfuric chloride, for example, phosphorus oxychloride, acetyl chloride, and thionyl chloride;
   (b) A compound of formula (VIII) is prepared from the compound of formula (IX) and ROH, preferably the compound of formula (VIII) is prepared from the compound of formula (IX) and ROH in the presence of CO, wherein R is C₁₋₆alkyl;
   (c) the compound of formula (VIII) and cyclopentyl formaldehyde are reacted to produce a compound of formula (VII);
   (d) 2-chloro-4-fluoro benzonitrile and hydrazine hydrate are reacted to produce a compound of formula (VI);
   (e) The compound of formula (VII) and the compound of formula (VI) are reacted to produce a compound of formula (V);
   (f) The compound of formula (V) is hydrolyzed to produce the compound of formula (IV);
   (g) The compound of formula (IV) and a chiral base Y are reacted to produce the compound of formula (III);
   (h) The compound of formula (III) is added to an acid solution and reacted to produce the compound of formula (II);
   (i) The compound of formula (II) and 4-hydroxylpiperidine are reacted to produce the compound of formula (I).
   Specific examples of reactions for Solution 9 can be referred to the following Aspects 1-12 of the present invention, particularly Aspect 10.
Solution 10: A process for preparing a compound of formula (I), comprising the following steps: 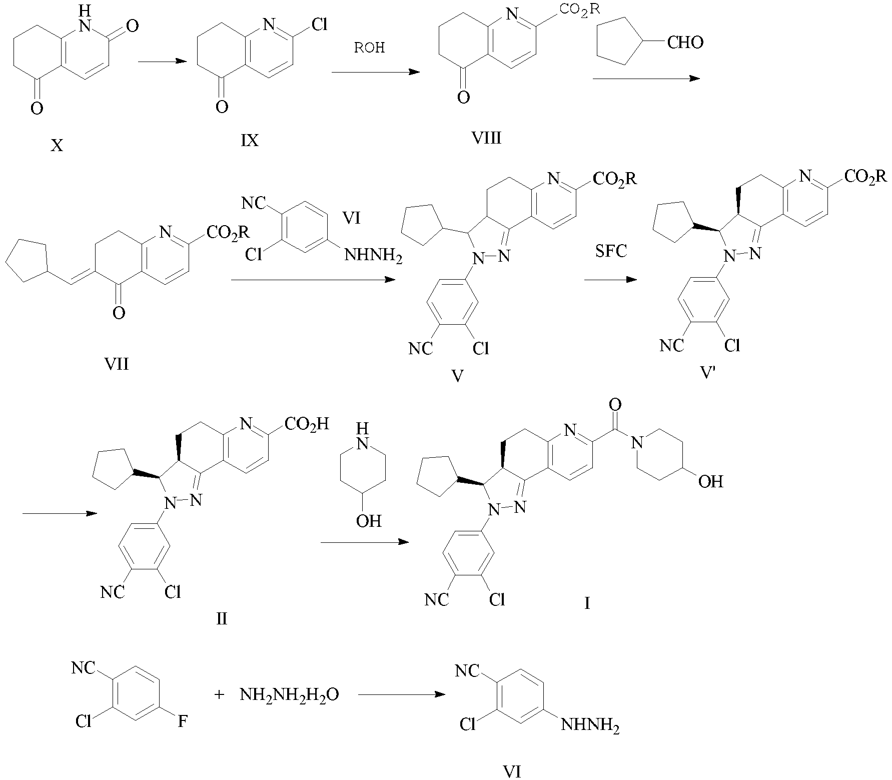
   (a) A compound of formula (IX) is prepared from a compound of formula (X), preferably the compound of formula (IX) is prepared from the compound of formula (X) and an acyl chloride compound, wherein the acyl chloride compound is selected from phosphoryl chloride, carbonyl chloride, and sulfuric chloride, for example, phosphorus oxychloride, acetyl chloride, and thionyl chloride;
   (b) A compound of formula (VIII) is prepared from the compound of formula (IX) and ROH, preferably the compound of formula (VIII) is prepared from the compound of formula (IX) and ROH in the presence of CO, wherein R is C₁₋₆alkyl;
   (c) the compound of formula (VIII) and cyclopentyl formaldehyde are reacted to produce a compound of formula (VII);
   (d) 2-chloro-4-fluoro benzonitrile and hydrazine hydrate are reacted to produce a compound of formula (VI);
   (e) The compound of formula (VII) and the compound of formula (VI) are reacted to produce a compound of formula (V);
   (f) The compound of formula (V) is resolved by SFC to produce a compound of formula (V');
   (g') the compound of formula (V') is hydrolyzed to produce a compound of formula (II);
   (h') the compound of formula (II) and 4-hydroxylpiperidine are reacted to produce the compound of formula (I).

Specific examples of reactions for Solution 10 can be referred to the following Aspects 1-12 of the present invention, particularly Aspect 12.

According to Aspect 1 of the present invention, is disclosed a process for producing a compound of formula (III) by the resolution of a compound of formula (IV).

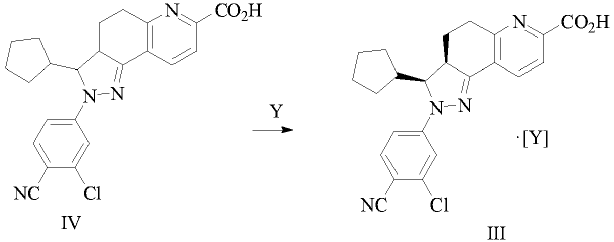

Said process comprises: reacting the compound of formula (IV) and a chiral base Y to produce the compound of formula (III), said process comprises a reaction in a ketone solvent, an ester solvent, or tetrahydrofuran; preferably, the reaction temperature is 10-70°C, for example, 10-50°C, 10-40°C, 20-30°C, e.g. 25°C, the reaction time is not less than 1 hour, for example, 1-48 hours, 4-24 hours, 8-16 hours, e.g. not less than 10 hours, the ratio of the compound of formula (IV) to the chiral base Y is less than or equal to 1:1.

Said chiral base Y is selected from (1S,2S)-cyclohexane diamine, (1R,2R)-cyclohexane diamine, quinine, quinidine, deuterated derivatives thereof, and the like, preferably (1S,2S)-cyclohexane diamine or (1R,2R)-cyclohexane diamine or deuterated derivatives thereof, more preferably (1S,2S)-cyclohexane diamine or deuterated derivatives thereof.

Said process comprises a reaction in a ketone solvent, an ester solvent, or tetrahydrofuran, said ketone solvent is selected from acetone, butanone, pentanone, methyl isobutyl ketone, and the like, preferably acetone; said ester solvent is selected from ethyl acetate, isopropyl acetate, tert-butyl acetate, and the like, preferably ethyl acetate.

The process of the present invention achieves the resolution of the isomers by adding the chiral base Y and compared with the supercritical liquid chromatography (SFC) resolution method in the prior art, it solves the limit that the batch size of the SFC method is too small, solves the expansion of large-scale industrial production, greatly reduces the production cost, and has high-resolution purity.

According to Aspect 2 of the present invention, is disclosed a process for producing a compound of formula (II).

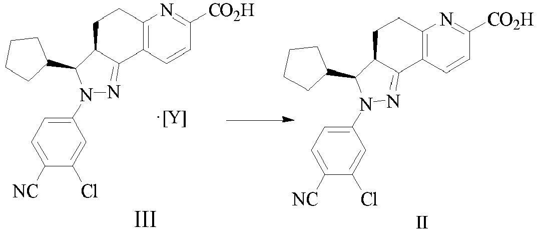

Said process comprises: mixing a compound of formula (III) with an alcohol solvent, adding an excessive acid solution, and reacting to produce the compound of formula (II); preferably, the reaction temperature is 0-40°C, for example, 20-30°C, e.g. 25°C, the reaction time is not less than 0.5 hours, for example, 0.5-10 hours, 1-5 hours, e.g. 2 hours.

Said alcohol solvent is selected from methanol, ethanol, isopropanol, tert-butanol, and the like. Said acid solution is selected from hydrochloric acid, sulfuric acid, phosphoric acid, nitric acid, trifluoroacetic acid, and the like.

According to Aspect 3 of the present invention, is disclosed a process for producing a compound of formula (I).

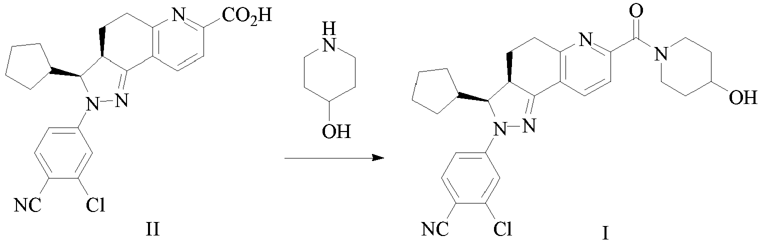

Said process comprises mixing a compound of formula (II), a condensation reagent, a polar solvent, and a non-polar solvent, then adding an organic base and 4-hydroxylpiperidine respectively, and reacting to produce the compound of formula (I). Preferably, the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours.

Said condensation reagent is selected from 2-(7-azabenzotriazol-1-yl)-N,N,N',N'-tetramethyluronium hexafluorophosphate (HATU), benzotriazolyl-N,N,N',N'-tetramethyluronium hexafluorophosphate (HBTU), O-benzotriazolyl-N,N,N',N'-tetramethyluronium tetrafluoroborate (TBTU), 1-(3-dimethylaminopropyl)-3-ethylcarbodiimide/1-hydroxylbenzotriazole (EDCI/HOBT), N,N'-dicyclohexylcarbodiimide/4-dimethylaminopyridine (DCC/DMAP), and the like, preferably 2-(7-azabenzotriazol-1-yl)-N,N,N',N'-tetramethyluronium hexafluorophosphate (HATU).

The polar solvent is selected from N-methyl pyrrolidinone (NMP), N,N-dimethyl formamide (DMF), N,N-dimethylacetamide (DMA), and the like, preferably N-methylpyrrolidinone (NMP). The non-polar solvent is selected from methylene chloride, tetrahydrofuran, 2-methyl tetrahydrofuran, chloroform, and the like, preferably methylene chloride.

Said organic base is selected from triethylamine, diethylamine, diisopropylethylamine (DIPEA), tetramethyl ethylene diamine, pyrrolidine, pyridine, 4-dimethylaminopyridine (DMAP), N-methyl morpholine (NMM), and the like, preferably triethylamine.

According to Aspect 4 of the present invention, is disclosed another process for preparing a compound of formula (I).

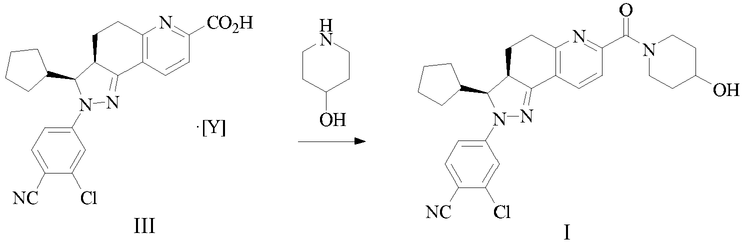

Said process comprises: mixing a compound of formula (III), a condensation reagent, a polar solvent and a non-polar solvent, then adding an organic base and 4-hydroxylpiperidine respectively, and reacting to produce the compound of formula (I). Preferably, the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours.

Said condensation reagent is selected from 2-(7-azabenzotriazol-1-yl)-N,N,N',N'-tetramethyluronium hexafluorophosphate (HATU), benzotriazolyl-N,N,N',N'-tetramethyluronium hexafluorophosphate (HBTU), O-benzotriazolyl-N,N,N',N'-tetramethyluronium tetrafluoroborate (TBTU), 1-(3-dimethylaminopropyl)-3-ethylcarbodiimide/1-hydroxylbenzotriazole (EDCI/HOBT), N,N'-dicyclohexylcarbodiimide/4-dimethylaminopyridine (DCC/DMAP), and the like, preferably 2-(7-azabenzotriazol-1-yl)-N,N,N',N'-tetramethyluronium hexafluorophosphate (HATU).

The polar solvent is selected from N-methylpyrrolidinone (NMP), N,N-dimethylformamide (DMF), N,N-dimethylacetamide (DMA), and the like, preferably N-methylpyrrolidinone (NMP). The non-polar solvent is selected from methylene chloride, tetrahydrofuran, 2-methyltetrahydrofuran, chloroform, and the like, preferably methylene chloride.

Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably triethylamine.

According to Aspect 5 of the present invention, is disclosed an intermediate compound of formula III, which has the following structure, and is useful in the preparation of the compound of formula (I) or the compound of formula (II).

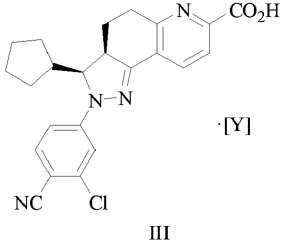

Said chiral base Y is selected from (1S,2S)-cyclohexane diamine, (1R,2R)-cyclohexane diamine, quinine, quinidine, deuterated derivatives thereof, and the like, preferably (1S,2S)-cyclohexane diamine or (1R,2R)-cyclohexane diamine or deuterated derivatives thereof, more preferably (1S,2S)-cyclohexane diamine or deuterated derivatives thereof.

According to Aspect 6 of the present invention, is disclosed a process for preparing a compound of formula (VIII).

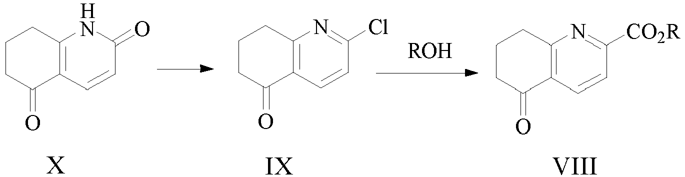

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce the compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF). Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably triethylamine.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃ or Pd(dba)₂.

WO2014094664 discloses a process for preparing the compound of formula (VIII), but the yield is low, the reactant m-chloroperoxybenzoic acid (mCPBA) has a high explosion risk, and the reactant trimethylsilyl cyanide is extremely toxic.

The above-mentioned process for preparing the compound of formula (VIII) not only improves the yield but also avoids using m-chloroperoxybenzoic acid (mCPBA) with high risk and trimethylsilyl cyanide which is a highly toxic reactant.

According to Aspect 7 of the present invention, is disclosed a process for preparing a compound of formula (IV).

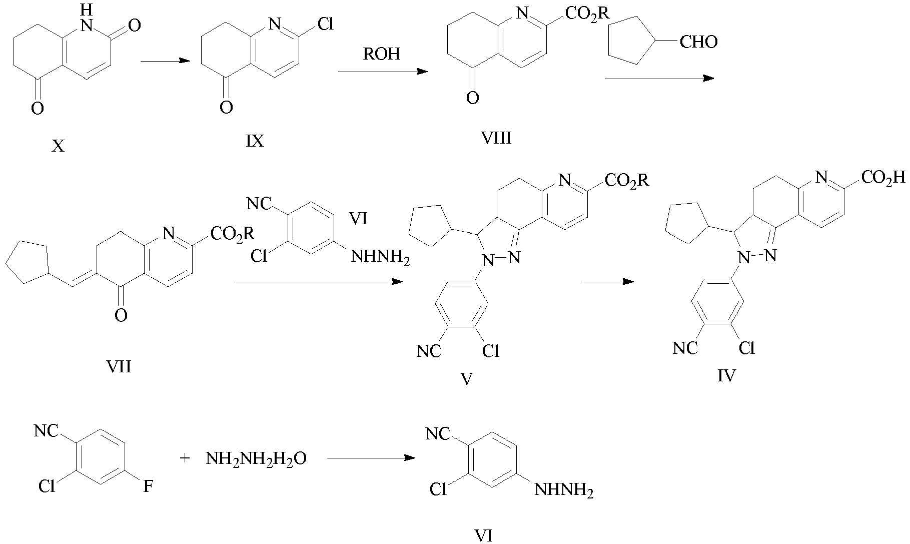

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce a compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours;

To the compound of formula (VIII) in an alcohol solvent, acetonitrile or THF is added cyclopentyl formaldehyde, then added an organic base, and reacted to produce a compound of formula (VII); preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;

2-chloro-4-fluorobenzonitrile in an alcohol solvent, acetonitrile or THF is mixed with hydrazine hydrate, and reacted to produce a compound of formula (VI); preferably, the reaction temperature is 40-90°C, for example, 75-85°C, e.g. 80°C, the reaction time is not less than 0.5 hours, for example, 0.5-15 hours, 1-5 hours, e.g. 3 hours;

The compound of formula (VII) and the compound of formula (VI) in an alcohol solvent, acetonitrile or THF and under a condition of hydrogen chloride are reacted to produce a compound of formula (V); preferably, the reaction temperature is 40-90°C, for example, 70-80°C, e.g. 75°C, the reaction time is not less than 3 hours, for example, 3-80 hours, 8-48 hours, 8-32 hours, e.g. 16 hours;

The compound of formula (V) is dissolved in a solution of an alcohol solvent and tetrahydrofuran, an inorganic base solution is added dropwise, and the resulting mixture is reacted to produce the compound of formula (IV); preferably, the reaction temperature is 0-40°C, for example, 0-20°C, 0-10°C, e.g. 5°C, the reaction time is not less than 0.1 hours, for example, 0.1-10 hours, for example, 0.1-5 hours, 0.5-2 hours, e.g. 1 hour.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF). Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.

Said alcohol solvent is selected from methanol, ethanol, isopropanol, tert-butanol, and the like, preferably ethanol.

Said inorganic base is selected from NaOH, KOH, LiOH, and the like, preferably NaOH or KOH. According to Aspect 8 of the present invention, is disclosed a process for preparing a compound of formula (III).

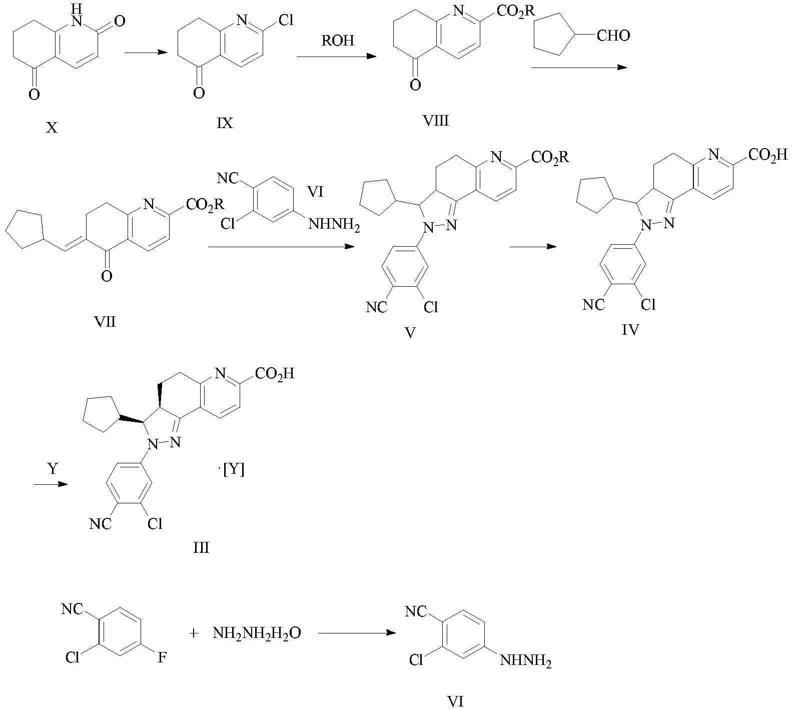

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce a compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours;

To the compound of formula (VIII) in an alcohol solvent, acetonitrile or THF is added cyclopentyl formaldehyde, then added an organic base, and reacted to produce a compound of formula (VII); preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;

2-chloro-4-fluorobenzonitrile in an alcohol solvent, acetonitrile or THF is mixed with hydrazine hydrate, and reacted to produce a compound of formula (VI); preferably, the reaction temperature is 40-90°C, for example, 75-85°C, e.g. 80°C, the reaction time is not less than 0.5 hours, for example, 0.5-15 hours, 1-5 hours, e.g. 3 hours;

The compound of formula (VII) and the compound of formula (VI) in an alcohol solvent, acetonitrile or THF and under a condition of hydrogen chloride are reacted to produce a compound of formula (V); preferably, the reaction temperature is 40-90°C, for example, 70-80°C, e.g. 75°C, the reaction time is not less than 3 hours, for example, 3-80 hours, 8-48 hours, 8-32 hours, e.g. 16 hours;

The compound of formula (V) is dissolved in a solution of an alcohol solvent and tetrahydrofuran, an inorganic base solution is added dropwise, and the resulting mixture is reacted to produce a compound of formula (IV); preferably, the reaction temperature is 0-40°C, for example, 0-20°C, 0-10°C, e.g. 5°C, the reaction time is not less than 0.1 hours, for example, 0.1-10 hours, 0.1-5 hours, 0.5-2 hours, e.g. 1 hour;

The compound of formula (IV) and a chiral base Y are reacted to produce the compound of formula (III), said process comprises a reaction in a ketone solvent, an ester solvent, or tetrahydrofuran; preferably, the reaction temperature is 10-70°C, for example, 10-50°C, 10-40°C, 20-30°C, e.g. 25°C, the reaction time is not less than 1 hour, for example, 1-48 hours, 4-24 hours, 8-16 hours, e.g. not less than 10 hours, the ratio of the compound of formula (IV) to the chiral base Y is less than or equal to 1:1.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF). Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.

Said alcohol solvent is selected from methanol, ethanol, isopropanol, tert-butanol, and the like, preferably ethanol.

Said inorganic base is selected from NaOH, KOH, LiOH, and the like, preferably NaOH or KOH. Said chiral resolution reagent is selected from (1S,2S)-cyclohexane diamine, (1R,2R)-cyclohexane diamine, quinine, quinidine, deuterated derivatives thereof, and the like, preferably (1S,2S)-cyclohexane diamine or (1R,2R)-cyclohexane diamine or deuterated derivatives thereof, more preferably (1S,2S)-cyclohexane diamine or deuterated derivatives thereof.

Said ketone solvent is selected from acetone, butanone, pentanone, methyl isobutyl ketone, and the like, preferably acetone.

Said ester solvent is selected from ethyl acetate, isopropyl acetate, tert-butyl acetate, and the like, preferably ethyl acetate.

According to Aspect 9 of the present invention, is disclosed a process for preparing a compound of formula (II).

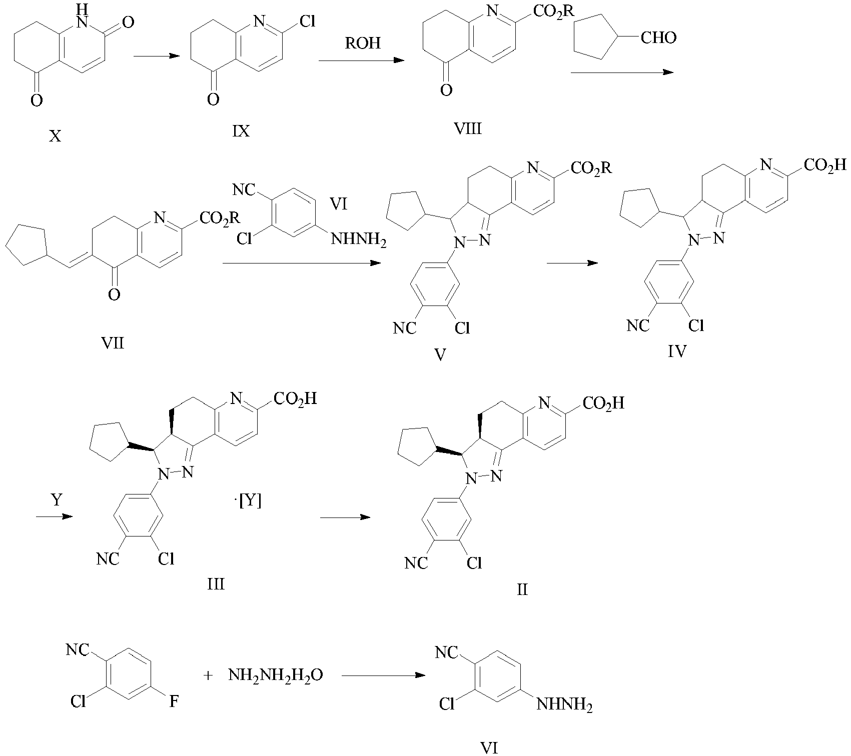

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce a compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours;

To the compound of formula (VIII) in an alcohol solvent, acetonitrile or THF is added cyclopentyl formaldehyde, then added an organic base, and reacted to produce a compound of formula (VII); preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;

2-chloro-4-fluorobenzonitrile in an alcohol solvent, acetonitrile or THF is mixed with hydrazine hydrate, and reacted to produce a compound of formula (VI); preferably, the reaction temperature is 40-90°C, for example, 75-85°C, e.g. 80°C, the reaction time is not less than 0.5 hours, for example, 0.5-15 hours, 1-5 hours, e.g. 3 hours;

The compound of formula (VII) and the compound of formula (VI) in an alcohol solvent, acetonitrile or THF and under a condition of hydrogen chloride are reacted to produce a compound of formula (V); preferably, the reaction temperature is 40-90°C, for example, 70-80°C, e.g. 75°C, the reaction time is not less than 3 hours, for example, 3-80 hours, 8-48 hours, 8-32 hours, e.g. 16 hours;

The compound of formula (V) is dissolved in a solution of an alcohol solvent and tetrahydrofuran, an inorganic base solution is added dropwise, and the resulting mixture is reacted to produce a compound of formula (IV); preferably, the reaction temperature is 0-40°C, for example, 0-20°C, 0-10°C, e.g. 5°C, the reaction time is not less than 0.1 hours, for example, 0.1-10 hours, for example, 0.1-5 hours, 0.5-2 hours, e.g. 1 hour;

The compound of formula (IV) and a chiral base Y are reacted to produce a compound of formula (III), said process comprises a reaction in a ketone solvent, an ester solvent, or tetrahydrofuran; preferably, the reaction temperature is 10-70°C, for example, 10-50°C, 10-40°C, 20-30°C, e.g. 25°C, the reaction time is not less than 1 hour, for example, 1-48 hours, 4-24 hours, 8-16 hours, e.g. not less than 10 hours, the ratio of the compound of formula (IV) to the chiral base Y is less than or equal to 1:1.

The compound of formula (III) is mixed with an alcohol solvent, an excessive acid solution is added, and the resulting mixture is reacted to produce a compound of formula (II); preferably, the reaction temperature is 0-40°C, for example, 20-30°C, e.g. 25°C, the reaction time is not less than 0.5 hours, for example, 0.5-10 hours, 1-5 hours, e.g. 2 hours.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF). Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.

Said alcohol solvent is selected from methanol, ethanol, isopropanol, tert-butanol, and the like, preferably ethanol.

Said inorganic base is selected from NaOH, KOH, LiOH, and the like, preferably NaOH or KOH. Said chiral resolution reagent is selected from (1S,2S)-cyclohexane diamine, (1R,2R)-cyclohexane diamine, quinine, quinidine, deuterated derivatives thereof, and the like, preferably (1S,2S)-cyclohexane diamine or (1R,2R)-cyclohexane diamine or deuterated derivatives thereof, more preferably (1S,2S)-cyclohexane diamine or deuterated derivatives thereof.

Said ketone solvent is selected from acetone, butanone, pentanone, methyl isobutyl ketone, and the like, preferably acetone.

Said ester solvent is selected from ethyl acetate, isopropyl acetate, tert-butyl acetate, and the like, preferably ethyl acetate.

Said acid solution is selected from hydrochloric acid, sulfuric acid, phosphoric acid, nitric acid, trifluoroacetic acid, and the like, preferably hydrochloric acid.

According to Aspect 10 of the present invention, is disclosed a process for preparing a compound of formula (I).

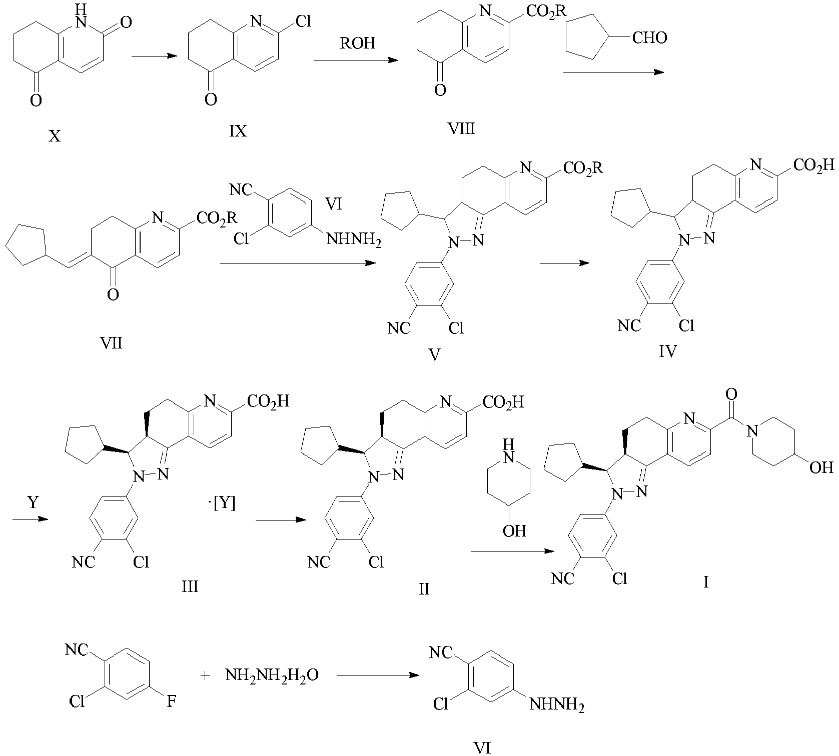

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce a compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours;

To the compound of formula (VIII) in an alcohol solvent, acetonitrile or THF is added cyclopentyl formaldehyde, then added an organic base, and reacted to produce a compound of formula (VII); preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;

2-chloro-4-fluorobenzonitrile in an alcohol solvent, acetonitrile or THF is mixed with hydrazine hydrate, and reacted to produce a compound of formula (VI); preferably, the reaction temperature is 40-90°C, for example, 75-85°C, e.g. 80°C, the reaction time is not less than 0.5 hours, for example, 0.5-15 hours, 1-5 hours, e.g. 3 hours;

The compound of formula (VII) and the compound of formula (VI) in an alcohol solvent, acetonitrile or THF and under a condition of hydrogen chloride are reacted to produce a compound of formula (V); preferably, the reaction temperature is 40-90°C, for example, 70-80°C, e.g. 75°C, the reaction time is not less than 3 hours, for example, 3-80 hours, 8-48 hours, 8-32 hours, e.g. 16 hours;

The compound of formula (V) is dissolved in a solution of an alcohol solvent and tetrahydrofuran, an inorganic base solution is added dropwise, and the resulting mixture is reacted to produce a compound of formula (IV); preferably, the reaction temperature is 0-40°C, for example, 0-20°C, 0-10°C, e.g. 5°C, the reaction time is not less than 0.1 hours, for example, 0.1-10 hours, 0.1-5 hours, 0.5-2 hours, e.g. 1 hour;

The compound of formula (IV) and a chiral base Y are reacted to produce a compound of formula (III), said process comprises a reaction in a ketone solvent, an ester solvent, or tetrahydrofuran; preferably, the reaction temperature is 10-70°C, for example, 10-50°C, 10-40°C, 20-30°C, e.g. 25°C, the reaction time is not less than 1 hour, for example, 1-48 hours, 4-24 hours, 8-16 hours, e.g. not less than 10 hours, the ratio of the compound of formula (IV) to the chiral base Y is less than or equal to 1:1.

The compound of formula (III) is mixed with an alcohol solvent, an excessive acid solution is added, and the resulting mixture is reacted to produce a compound of formula (II); preferably, the reaction temperature is 0-40°C, for example, 20-30°C, e.g. 25°C, the reaction time is not less than 0.5 hours, for example, 0.5-10 hours, 1-5 hours, e.g. 2 hours.

The compound of formula (II), a condensation reagent, a polar solvent, and a non-polar solvent are mixed, then an organic base and 4-hydroxylpiperidine are added respectively, and the resulting mixture is reacted to produce the compound of formula (I). Preferably, the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF), N-methylpyrrolidinone (NMP).

Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.

Said alcohol solvent is selected from methanol, ethanol, isopropanol, tert-butanol, and the like, preferably ethanol.

Said inorganic base is selected from NaOH, KOH, LiOH, and the like, preferably NaOH or KOH. Said chiral resolution reagent is selected from (1S,2S)-cyclohexane diamine, (1R,2R)-cyclohexane diamine, quinine, quinidine, deuterated derivatives thereof, and the like, preferably (1S,2S)-cyclohexane diamine or (1R,2R)-cyclohexane diamine or deuterated derivatives thereof, more preferably (1S,2S)-cyclohexane diamine or deuterated derivatives thereof.

Said ketone solvent is selected from acetone, butanone, pentanone, methyl isobutyl ketone, and the like, preferably acetone.

Said ester solvent is selected from ethyl acetate, isopropyl acetate, tert-butyl acetate, and the like, preferably ethyl acetate.

Said acid solution is selected from hydrochloric acid, sulfuric acid, phosphoric acid, nitric acid, trifluoroacetic acid, and the like, preferably hydrochloric acid.

Said condensation reagent is selected from 2-(7-azabenzotriazol-1-yl)-N,N,N',N'-tetramethyluronium hexafluorophosphate (HATU), benzotriazolyl-N,N,N',N'-tetramethyluronium hexafluorophosphate (HBTU), O-benzotriazolyl-N,N,N',N'-tetramethyluronium tetrafluoroborate (TBTU), 1-(3-dimethylaminopropyl)-3-ethylcarbodiimide/1-hydroxylbenzotriazole (EDCI/HOBT), N,N'-dicyclohexylcarbodiimide/4-dimethylaminopyridine (DCC/DMAP), and the like, preferably 2-(7-azabenzotriazol-1-yl)-N,N,N',N'-tetramethyluronium hexafluorophosphate (HATU).

The non-polar solvent is selected from methylene chloride, tetrahydrofuran, 2-methyl tetrahydrofuran, chloroform and the like, preferably methylene chloride.

According to Aspect 11 of the present invention, is disclosed a process for preparing a compound of formula (I).

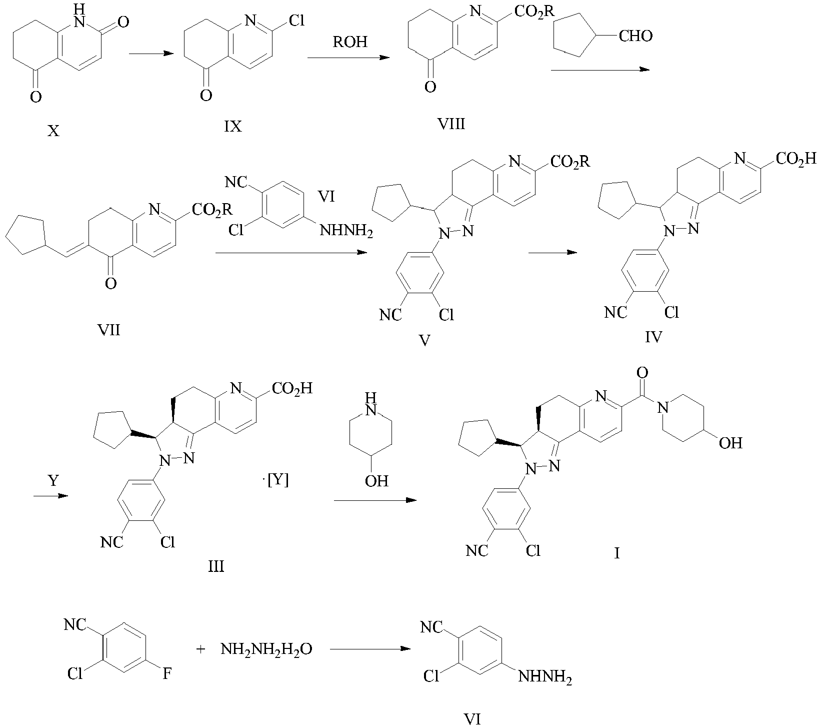

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce a compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours;

To the compound of formula (VIII) in an alcohol solvent, acetonitrile or THF is added cyclopentyl formaldehyde, then added an organic base, and reacted to produce a compound of formula (VII); preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;

2-chloro-4-fluorobenzonitrile in an alcohol solvent, acetonitrile or THF is mixed with hydrazine hydrate, and reacted to produce a compound of formula (VI); preferably, the reaction temperature is 40-90°C, for example, 75-85°C, e.g. 80°C, the reaction time is not less than 0.5 hours, for example, 0.5-15 hours, 1-5 hours, e.g. 3 hours;

The compound of formula (VII) and the compound of formula (VI) in an alcohol solvent, acetonitrile or THF and under a condition of hydrogen chloride are reacted to produce a compound of formula (V); preferably, the reaction temperature is 40-90°C, for example, 70-80°C, e.g. 75°C, the reaction time is not less than 3 hours, for example, 3-80 hours, 8-48 hours, 8-32 hours, e.g. 16 hours;

The compound of formula (V) is dissolved in a solution of an alcohol solvent and tetrahydrofuran, an inorganic base solution is added dropwise, and the resulting mixture is reacted to produce a compound of formula (IV); preferably, the reaction temperature is 0-40°C, for example, 0-20°C, 0-10°C, e.g. 5°C, the reaction time is not less than 0.1 hours, for example, 0.1-10 hours, 0.1-5 hours, 0.5-2 hours, e.g. 1 hour;

The compound of formula (IV) and a chiral base Y are reacted to produce a compound of formula (III), said process comprises a reaction in a ketone solvent, an ester solvent, or tetrahydrofuran; preferably, the reaction temperature is 10-70°C, for example, 10-50°C, 10-40°C, 20-30°C, e.g. 25°C, the reaction time is not less than 1 hour, for example, 1-48 hours, 4-24 hours, 8-16 hours, e.g. not less than 10 hours, the ratio of the compound of formula (IV) to the chiral base Y is less than or equal to 1:1.

The compound of formula (III), a condensation reagent, a polar solvent, and a non-polar solvent are mixed, then an organic base and 4-hydroxylpiperidine are added respectively, and the resulting mixture is reacted to produce the compound of formula (I). Preferably, the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF), N-methylpyrrolidinone (NMP).

Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.

Said alcohol solvent is selected from methanol, ethanol, isopropanol, tert-butanol, and the like, preferably ethanol.

Said inorganic base is selected from NaOH, KOH, LiOH, and the like, preferably NaOH or KOH. Said chiral resolution reagent is selected from (1S,2S)-cyclohexane diamine, (1R,2R)-cyclohexane diamine, quinine, quinidine, deuterated derivatives thereof, and the like, preferably (1S,2S)-cyclohexane diamine or (1R,2R)-cyclohexane diamine or deuterated derivatives thereof, more preferably (1S,2S)-cyclohexane diamine or deuterated derivatives thereof.

Said ketone solvent is selected from acetone, butanone, pentanone, methyl isobutyl ketone, and the like, preferably acetone.

Said ester solvent is selected from ethyl acetate, isopropyl acetate, tert-butyl acetate, and the like, preferably ethyl acetate.

Said condensation reagent is selected from 2-(7-azabenzotriazol-1-yl)-N,N,N',N'-tetramethyluronium hexafluorophosphate (HATU), benzotriazolyl-N,N,N',N'-tetramethyluronium hexafluorophosphate (HBTU), O-benzotriazolyl-N,N,N',N'-tetramethyluronium tetrafluoroborate (TBTU), 1-(3-dimethylaminopropyl)-3-ethylcarbodiimide/1-hydroxylbenzotriazole (EDCI/HOBT), N,N'-dicyclohexylcarbodiimide/4-dimethylaminopyridine (DCC/DMAP), and the like, preferably 2-(7-azabenzotriazol-1-yl)-N,N,N',N'-tetramethyluronium hexafluorophosphate (HATU).

The non-polar solvent is selected from methylene chloride, tetrahydrofuran, 2-methyltetrahydrofuran, chloroform and the like, preferably methylene chloride.

According to Aspect 12 of the present invention, is disclosed a process for preparing a compound of formula (I).

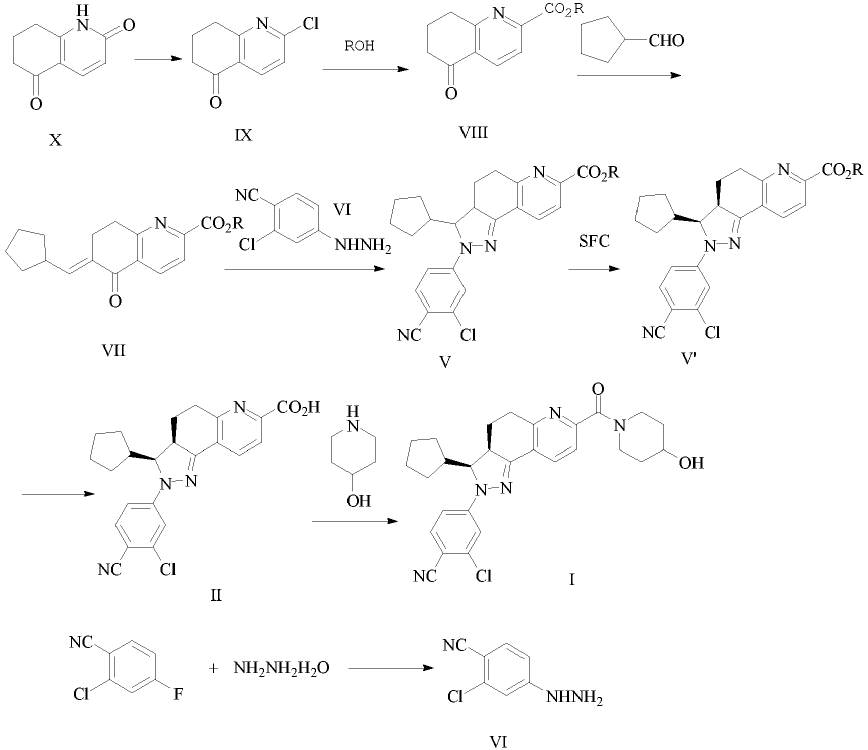

### Said process comprises:

A compound of formula (X) and an acyl chloride compound are reacted to produce a compound of formula (IX), preferably the compound of formula (X) and phosphorus oxychloride are reacted to produce the compound of formula (IX); preferably, the reaction temperature is 40-90°C, for example, 75-85°C, e.g. 80°C, the reaction time is not less than 0.5 hours, for example, 0.5-10 hours, 1-5 hours, e.g. 2 hours.

To the compound of formula (IX) in ROH, a polar solvent, an organic base, and a catalyst is introduced carbon monoxide, and reacted to produce a compound of formula (VIII); preferably, the reaction temperature is 40-90°C, for example, 70-80°C, e.g. 75°C, the reaction time is not less than 0.5 hours, for example, 0.5-15 hours, 1-5 hours, e.g. 3 hours;

To the compound of formula (VIII) in an alcohol solvent, acetonitrile or THF is added cyclopentyl formaldehyde, then added an organic base, and reacted to produce a compound of formula (VII); preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;

2-chloro-4-fluorobenzonitrile in an alcohol solvent, acetonitrile or THF is mixed with hydrazine hydrate, and reacted to produce a compound of formula (VI); preferably, the reaction temperature is 40-90°C, for example, 75-85°C, e.g. 80°C, the reaction time is not less than 0.5 hours, for example, 0.5-15 hours, 1-5 hours, e.g. 3 hours;

The compound of formula (VII) and the compound of formula (VI) in an alcohol solvent, acetonitrile or THF and under a condition of hydrogen chloride are reacted to produce a compound of formula (V); preferably, the reaction temperature is 40-90°C, for example, 70-80°C, e.g. 75°C, the reaction time is not less than 3 hours, for example, 3-80 hours, 8-48 hours, 8-32 hours, e.g. 16 hours;

The compound of formula (V) is resolved by SFC to produce a compound of formula (V');

The compound of formula (V') is dissolved in a solution of an alcohol solvent and tetrahydrofuran, an inorganic base solution is added dropwise, and the resulting mixture is reacted to produce a compound of formula (II);

The compound of formula (II), a condensation reagent, a polar solvent and, a non-polar solvent are mixed, then an organic base and 4-hydroxylpiperidine are added respectively, and the resulting mixture is reacted to produce the compound of formula (I). Preferably, the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours.

R represents C₁₋₆alkyl, which refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Said acyl chloride compound is selected from phosphoryl chloride, carbonyl chloride, and sulfuric chloride, e.g. phosphorus oxychloride, acetyl chloride, and thionyl chloride.

The polar solvent is selected from N-methylpyrrolidinone (NMP), N,N-dimethylformamide (DMF), N,N-dimethylacetamide (DMA), and the like, preferably N,N-dimethylformamide (DMF), N-methylpyrrolidinone (NMP).

Said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like.

The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.

Said alcohol solvent is selected from methanol, ethanol, isopropanol, tert-butanol, and the like, preferably ethanol.

Said inorganic base is selected from NaOH, KOH, LiOH, and the like, preferably NaOH or KOH. Unless otherwise indicated, any step of the preparation process of the present invention is carried out under normal pressure.

According to the present invention, room temperature refers to 10-30°C, for example, 26°C. Specifically, the present invention provides the following embodiments:
1. A process for preparing a compound of formula (III), wherein the compound of formula (III) is obtained through the following step (g):
   (g) reacting a compound of formula (IV) and a chiral base Y to produce the compound of formula (III), 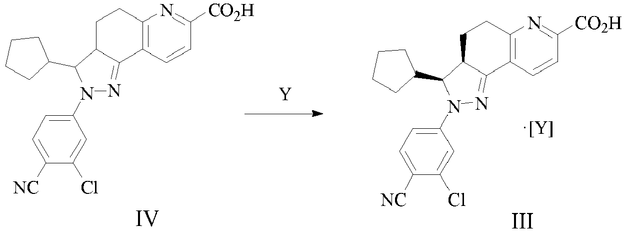 said chiral base Y is selected from (1S,2S)-cyclohexane diamine, (1R,2R)-cyclohexane diamine, quinine, quinidine or deuterated derivatives thereof, preferably (1S,2S)-cyclohexane diamine or (1R,2R)-cyclohexane diamine or deuterated derivatives thereof, more preferably (1S,2S)-cyclohexane diamine or deuterated derivatives thereof.
2. The process of Embodiment 1, wherein said step (g) is a reaction in a ketone solvent, an ester solvent, or tetrahydrofuran;
   Preferably, the reaction temperature is 10-70°C, for example, 10-50°C, 10-40°C, 20-30°C, e.g. 25°C; the reaction time is not less than 1 hour, for example, 1-48 hours, 4-24 hours, 8-16 hours, e.g. not less than 10 hours;
   More preferably, the ratio of the compound of formula (IV) to the chiral base Y is less than or equal to 1:1;
   Further more preferably, said ketone solvent is selected from acetone, butanone, pentanone, methyl isobutyl ketone, and the like, preferably acetone; said ester solvent is selected from ethyl acetate, isopropyl acetate, tert-butyl acetate, and the like, preferably ethyl acetate.
3. The process of Embodiment 1 or 2, wherein the compound of formula (IV) is obtained by the following step (f):
   (f) A compound of formula (V) is hydrolyzed to produce the compound of formula (IV) 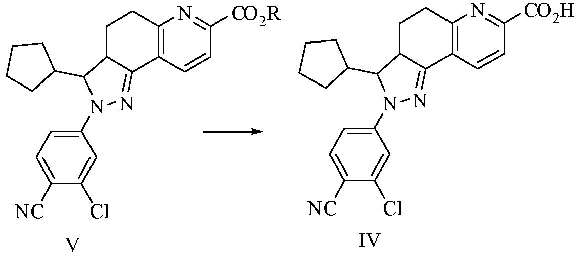 wherein R is C₁₋₆alkyl;
   Preferably, step (f) comprises the compound of formula (V) is dissolved in a solution of an alcohol solvent and tetrahydrofuran, an inorganic base solution is added dropwise, and the resulting mixture is reacted to produce the compound of formula (IV);
   More preferably, the reaction temperature is 0-40°C, for example, 0-20°C, 0-10°C, e.g. 5°C, the reaction time is not less than 0.1 hours, for example, 0.1-10 hours, 0.1-5 hours, 0.5-2 hours, e.g. 1 hour;
   Further preferably, said alcohol solvent is selected from methanol, ethanol, isopropanol, tert-butanol, and the like, preferably ethanol; said inorganic base is selected from NaOH, KOH, LiOH, and the like, preferably NaOH or KOH.
4. The process of Embodiment 3, wherein said compound of formula (V) is obtained by the following step (e):
   (e) A compound of formula (VII) and a compound of formula (VI) are reacted to produce the compound of formula (V) 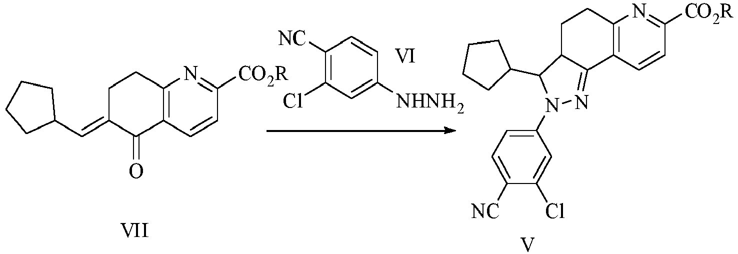 wherein R is C₁₋₆alkyl;
   Preferably, step (e) comprises the compound of formula (VII) and the compound of formula (VI) in an alcohol solvent, acetonitrile or THF and under a condition of hydrogen chloride are reacted to produce the compound of formula (V);
   More preferably, the reaction temperature is 40-90°C, for example, 70-80°C, 75°C, the reaction time is not less than 3 hours, for example, 3-80 hours, 8-48 hours, 8-32 hours, e.g. 16 hours; Further preferably, said alcohol solvent is selected from methanol, ethanol, isopropanol, tert-butanol, and the like, preferably ethanol.
5. The process of Embodiment 4, wherein said compound of formula (VI) is obtained by the following step (d):
   (d) 2-chloro-4-fluoro benzonitrile and hydrazine hydrate are reacted to produce the compound of formula (VI) 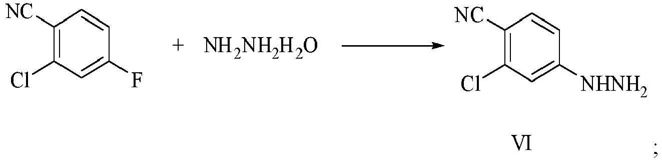
   Preferably, step (d) comprises: 2-chloro-4-fluorobenzonitrile in an alcohol solvent, acetonitrile or THF is mixed with hydrazine hydrate, and reacted to produce the compound of formula (VI); More preferably, the reaction temperature is 40-90°C, for example, 75-85°C, e.g. 80°C, the reaction time is not less than 0.5 hours, for example, 0.5-15 hours, 1-5 hours, e.g. 3 hours; Further preferably, said alcohol solvent is selected from methanol, ethanol, isopropanol, tert-butanol, and the like, preferably ethanol.
6. The process of Embodiment 4, wherein said compound of formula (VII) is obtained by the following step (c):
   (c) A compound of formula (VIII) and cyclopentyl formaldehyde are reacted to produce a compound of formula (VII) 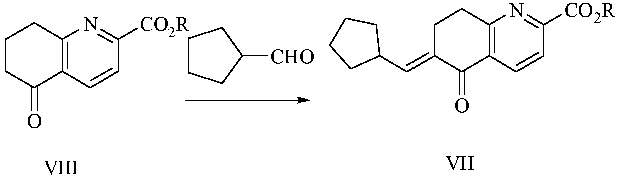 wherein R is C₁₋₆alkyl;
   Preferably, step (c) comprises: to the compound of formula (VIII) in an alcohol solvent, acetonitrile or THF is added cyclopentyl formaldehyde, then added an organic base, and reacted to produce the compound of formula (VII);
   More preferably, the reaction temperature is 0-40°C, for example, 10-30°C, e.g. 25°C, the reaction time is not less than 2 hours, for example, 2-40 hours, 4-24 hours, 4-16 hours, e.g. 8 hours;
   Further preferably, said alcohol solvent is selected from methanol, ethanol, isopropanol, tert-butanol, and the like, preferably ethanol; said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably pyrrolidine.
7. The process of Embodiment 6, wherein said compound of formula (VIII) is obtained by the following step (b):
   (b) The compound of formula (VIII) is prepared from a compound of formula (IX) and ROH, preferably the compound of formula (VIII) is prepared from the compound of formula (IX) and ROH in the presence of CO 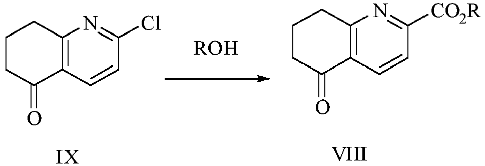 wherein R is C₁₋₆alkyl;
   Preferably, step (b) comprises: to the compound of formula (IX) in ROH, a polar solvent, an organic base, and a catalyst is introduced carbon monoxide, and reacted to produce the compound of formula (VIII);
   More preferably, the reaction temperature is 40-90°C, for example, 70-80°C, e.g. 75°C, the reaction time is not less than 0.5 hours, for example, 0.5-15 hours, 1-5 hours, e.g. 3 hours; Further preferably, the polar solvent is selected from N-methylpyrrolidinone (NMP), N,N-dimethylformamide (DMF), N,N-dimethylacetamide (DMA), and the like, preferably N,N-dimethylformamide (DMF); said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably triethylamine; The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.
8. The process of Embodiment 7, wherein said compound of formula (IX) is obtained by the following step (a):
   (a) The compound of formula (IX) is prepared from a compound of formula (X), preferably the compound of formula (IX) is prepared from the compound of formula (X) and an acyl chloride compound 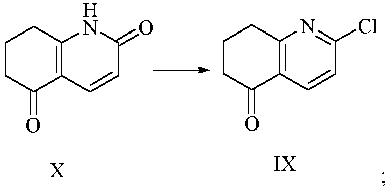
   Preferably, the acyl chloride compound is selected from phosphoryl chloride, carbonyl chloride, and sulfuric chloride, e.g. phosphorus oxychloride, acetyl chloride, and thionyl chloride;
   More preferably, the reaction temperature is 40-90°C, for example, 75-85°C, e.g. 80°C, the reaction time is not less than 0.5 hours, for example, 0.5-10 hours, 1-5 hours, e.g. 2 hours.
9. The process of Embodiment 6, wherein said compound of formula (VIII) is obtained by the following steps (a) and (b):
   (a) A compound of formula (IX) is prepared from a compound of formula (X), preferably the compound of formula (IX) is prepared from the compound of formula (X) and an acyl chloride compound,
   (b) The compound of formula (VIII) is prepared from the compound of formula (IX) and ROH, preferably the compound of formula (VIII) is prepared from the compound of formula (IX) and ROH in the presence of CO, 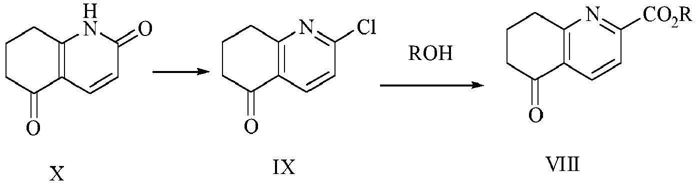 wherein R is C₁₋₆alkyl;

   Preferably, step (a) comprises: the compound of formula (X) and an acyl chloride compound are reacted to produce the compound of formula (IX), preferably the compound of formula (X) and phosphorus oxychloride are reacted to produce the compound of formula (IX); wherein the acyl chloride compound is selected from phosphoryl chloride, carbonyl chloride, and sulfuric chloride, e.g. phosphorus oxychloride, acetyl chloride, and thionyl chloride; step (b) comprises: to the compound of formula (IX) in ROH, a polar solvent, an organic base, and a catalyst is introduced carbon monoxide and reacted to produce the compound of formula (VIII);
   More preferably, step (a): the reaction temperature is 40-90°C, for example, 75-85°C, e.g. 80°C, the reaction time is not less than 0.5 hours, for example, 0.5-10 hours, 1-5 hours, e.g. 2 hours; step (b): the reaction temperature is 40-90°C, for example, 70-80°C, e.g. 75°C, the reaction time is not less than 0.5 hours, for example, 0.5-15 hours, 1-5 hours, e.g. 3 hours;
   Further preferably, the polar solvent is selected from N-methylpyrrolidinone (NMP), N,N-dimethylformamide (DMF), N,N-dimethylacetamide (DMA), and the like, preferably N,N-dimethylformamide (DMF); said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably triethylamine; The catalyst is selected from palladium catalysts, including Pd(dppf)Cl₂•CH₂Cl₂, Pd(dppf)Cl₂, Pd(PPh₃)₄, Pd(PPh₃)₂Cl₂, Pd(OAc)₂, Pd₂(dba)₃, Pd(dba)₂, and the like, preferably Pd(dppf)Cl₂•CH₂Cl₂.
10. The process of any of Embodiments 1-8, wherein the process also comprises using the compound of formula (III) to prepare a compound of formula (II), which comprises the following steps:
   (h) The compound of formula (III) is added to an acid solution and reacted to produce the compound of formula (II), 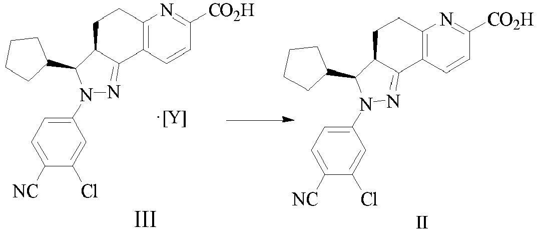
   wherein Y is selected from (1S,2S)-cyclohexane diamine, (1R,2R)-cyclohexane diamine, quinine, quinidine or deuterated derivatives thereof;
   Preferably, step (h) comprises: the compound of formula (III) is mixed with an alcohol solvent, an excessive acid solution is added, and the resulting mixture is reacted to produce the compound of formula (II);
   More preferably, the reaction temperature is 0-40°C, for example, 20-30°C, e.g. 25°C, the reaction time is not less than 0.5 hours, for example, 0.5-10 hours, 1-5 hours, e.g. 2 hours;
   Further preferably, said alcohol solvent is selected from methanol, ethanol, isopropanol, tert-butanol, and the like, preferably ethanol; said acid solution is selected from hydrochloric acid, sulfuric acid, phosphoric acid, nitric acid, trifluoroacetic acid, and the like, preferably hydrochloric acid.
11. The process of any of Embodiments 1-8, wherein the process also comprises using the compound of formula (III) to prepare a compound of formula (I), which comprises the following steps: 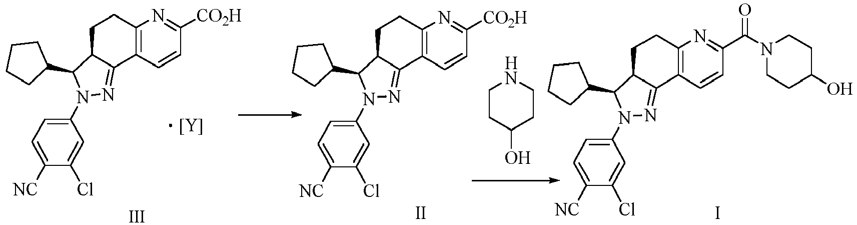
   (h) The compound of formula (III) is added to an acid solution and reacted to produce a compound of formula (II);
   (i) The compound of formula (II) and 4-hydroxylpiperidine are reacted to produce the compound of formula (I), wherein Y is selected from (1S,2S)-cyclohexane diamine, (1R,2R)-cyclohexane diamine, quinine, quinidine or deuterated derivatives thereof;
   Preferably, step (h) comprises: the compound of formula (III) is mixed with an alcohol solvent, an excessive acid solution is added, and the resulting mixture is reacted to produce the compound of formula (II); step (i) comprises: the compound of formula (II), a condensation reagent, a polar solvent, and a non-polar solvent are mixed, then an organic base and 4-hydroxylpiperidine are added respectively, and the resulting mixture is reacted to produce the compound of formula (I); More preferably, step (h): the reaction temperature is 0-40°C, for example, 20-30°C, e.g. 25°C, the reaction time is not less than 0.5 hours, for example, 0.5-10 hours, 1-5 hours, e.g. 2 hours; step (i): the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours;
   Further preferably, said alcohol solvent is selected from methanol, ethanol, isopropanol, tert-butanol, and the like, preferably ethanol; said acid solution is selected from hydrochloric acid, sulfuric acid, phosphoric acid, nitric acid, trifluoroacetic acid, and the like, preferably hydrochloric acid; said condensation reagent is selected from 2-(7-azabenzotriazol-1-yl)-N,N,N',N'-tetramethyluronium hexafluorophosphate (HATU), benzotriazolyl-N,N,N',N'-tetramethyluronium hexafluorophosphate (HBTU), O-benzotriazolyl-N,N,N',N'-tetramethyluronium tetrafluoroborate (TBTU), 1-(3-dimethylaminopropyl)-3-ethylcarbodiimide/1-hydroxylbenzotriazole (EDCI/HOBT), N,N'-dicyclohexylcarbodiimide/4-dimethylaminopyridine (DCC/DMAP), and the like, preferably 2-(7-azabenzotriazol-1-yl)-N,N,N',N'-tetramethyluronium hexafluorophosphate (HATU); the polar solvent is selected from N-methylpyrrolidinone (NMP), N,N-dimethylformamide (DMF), N,N-dimethylacetamide (DMA), and the like, preferably N-methylpyrrolidinone (NMP); the non-polar solvent is selected from methylene chloride, tetrahydrofuran, 2-methyltetrahydrofuran, chloroform, and the like, preferably methylene chloride; said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably triethylamine.
12. The process of any of Embodiments 1-8, wherein the process also comprises using the compound of formula (III) to prepare a compound of formula (I), which comprises the following steps:
   The compound of formula (III) and 4-hydroxylpiperidine are reacted to produce the compound of formula (I), 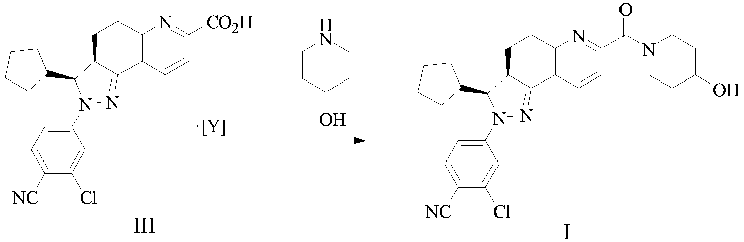 wherein Y is selected from (1S,2S)-cyclohexane diamine, (1R,2R)-cyclohexane diamine, quinine, quinidine or deuterated derivatives thereof;
   Preferably, the above-mentioned step comprises: the compound of formula (III), a condensation reagent, a polar solvent, and a non-polar solvent are mixed, then an organic base and 4-hydroxylpiperidine are added respectively, and the resulting mixture is reacted to produce the compound of formula (I);
   More preferably, in the above-mentioned step: the reaction temperature is 0-40°C, for example, 20-30°C, e.g. 25°C, the reaction time is not less than 0.2 hours, for example, 0.2-20 hours, 0.5-10 hours, 1-5 hours, e.g. 2 hours;
   Further preferably, said condensation reagent is selected from 2-(7-azabenzotriazol-1-yl)-N,N,N',N'-tetramethyluronium hexafluorophosphate (HATU), benzotriazolyl-N,N,N',N'-tetramethyluronium hexafluorophosphate (HBTU), O-benzotriazolyl-N,N,N',N'-tetramethyluronium tetrafluoroborate (TBTU), 1-(3-dimethylaminopropyl)-3-ethylcarbodiimide/1-hydroxylbenzotriazole (EDCI/HOBT), N,N'-dicyclohexylcarbodiimide/4-dimethylaminopyridine (DCC/DMAP), and the like, preferably 2-(7-azabenzotriazol-1-yl)-N,N,N',N'-tetramethyluronium hexafluorophosphate (HATU);
   The polar solvent is selected from N-methylpyrrolidinone (NMP), N,N-dimethylformamide (DMF), N,N-dimethylacetamide (DMA), and the like, preferably N-methylpyrrolidinone (NMP); the non-polar solvent is selected from methylene chloride, tetrahydrofuran, 2-methyltetrahydrofuran, chloroform, and the like, preferably methylene chloride;
      said organic base is selected from triethylamine, diethylamine, diisopropylethylamine (DIPEA), tetramethyl ethylene diamine, pyrrolidine, pyridine, 4-dimethylaminopyridine (DMAP), N-methylmorpholine (NMM), and the like, preferably triethylamine.
13. A compound of formula (III), which has the following structure: 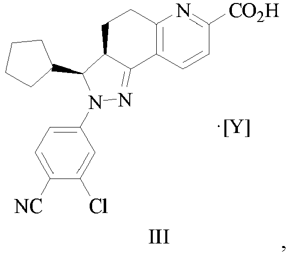 wherein Y is selected from (1S,2S)-cyclohexane diamine, (1R,2R)-cyclohexane diamine, quinine, quinidine, or deuterated derivatives thereof.

In the present invention, including in any of the above-mentioned embodiments, C₁₋₆alkyl refers to a linear or branched alkyl derived by removing one hydrogen atom from an alkane containing 1-6 carbon atoms, e.g. methyl, ethyl, n-propyl, iso-propyl, n-butyl, iso-butyl, sec-butyl, tert-butyl, n-pentyl, iso-pentyl, 2-methylbutyl, neo-pentyl, 1-ethylpropyl, n-hexyl, iso-hexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1-methyl-2-methylpropyl, and the like.

Especially, the present invention also provides the following technical solutions:
Technical Solution 1: A process for preparing (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt, which comprises the following steps:

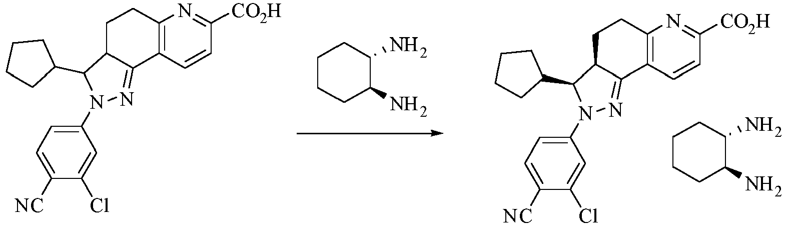

At 10-40°C (for example, 20-30°C, e.g. 25°C), 2-(3-chloro-4-cyano-phenyl)-3 -cyclopentyl-3,3 a,4,5 -tetrahydro-2H-pyrazolo[3,4-f]quinoline-7-car boxylic acid (1 molar equivalent) and (1S,2S)-cyclohexane diamine (not less than 0.4 molar equivalents, for example, 0.4-10 molar equivalents, 1-4 molar equivalents, e.g. 2 molar equivalents) are added to acetone (not less than 8.42 L/1 molar equivalent, for example 8.42-210.45 L/1 molar equivalent, 21.04-84.18 L/1 molar equivalent, e.g. 42089 mL/1 molar equivalent), the resulting mixture is stirred at 10-70°C (for example, 10-50°C, 10-40°C, 20-30°C, e.g. 25°C) for not less than 1 hour (for example 1-48 hours, 4-24 hours, 8-16 hours, e.g. not less than 10 hours), filtered, and the filter cake is washed with acetone to produce (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt.

Technical Solution 2: A process for preparing (3S,3aR)-2-(3-chloro-4-cyano-phenyl)-3-cyclopentyl-3,3a,4,5-tetrahydro-2H-pyrazolo[3,4-f]quino line-7-carboxylic acid (1R,2R)-cyclohexane diamine salt, which comprises the following steps:

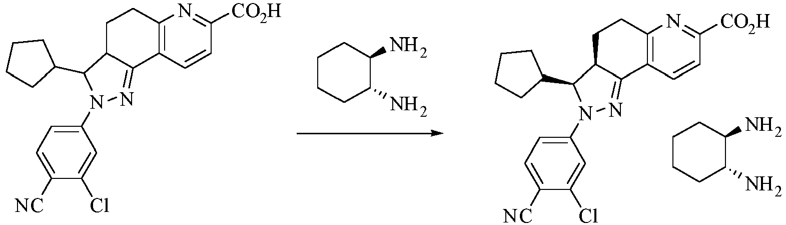

At 10-40°C (for example, 20-30°C, e.g. 25°C), 2-(3-chloro-4-cyano-phenyl)-3 -cyclopentyl-3,3 a,4,5 -tetrahydro-2H-pyrazolo[3,4-f]quinoline-7-car boxylic acid (1 molar equivalent) and (1R,2R)-cyclohexane diamine (not less than 0.4 molar equivalents, for example, 0.4-10 molar equivalents, 1-4 molar equivalents, e.g. 2 molar equivalents) are added to acetone (not less than 8.42 L/1 molar equivalent, for example 8.42-210.45 L/1 molar equivalent, 21.04-84.18 L/1 molar equivalent, e.g. 42089 mL/1 molar equivalent), the resulting mixture is stirred at 10-70°C (for example, 10-50°C, 10-40°C, 20-30°C, e.g. 25°C) for not less than 1 hour (for example 1-48 hours, 4-24 hours, 8-16 hours, e.g. not less than 10 hours), filtered, and the collected mother liquor is distilled to produce (3S,3aR)-2-(3-chloro-4-cyano-phenyl)-3-cyclopentyl-3,3a,4,5-tetrahydro-2H-pyrazolo[3,4-f]quino line-7-carboxylic acid (1R,2R)-cyclohexane diamine salt.

Technical Solution 3: A process for preparing (3S,3aR)-2-(3-chloro-4-cyano-phenyl)-3-cyclopentyl-3,3a,4,5-tetrahydro-2H-pyrazolo[3,4-f]quino line-7-carboxylic acid quinine salt, which comprises the following steps:
At 10-40°C (for example, 20-30°C, e.g. 25°C), 2-(3-chloro-4-cyano-phenyl)-3 -cyclopentyl-3,3 a,4,5 -tetrahydro-2H-pyrazolo[3,4-f]quinoline-7-car boxylic acid (1 molar equivalent) and quinine (not less than 0.2 molar equivalents, for example, 0.2-5 molar equivalents, 0.5-2 molar equivalents, e.g. 1 molar equivalent) are aded to ethyl acetate (not less than 5.05 L/1 molar equivalent, for example 5.05-126.27 L/1 molar equivalent, for example 12.63-50.51 L/1 molar equivalent, e.g. 25253.4 mL/1 molar equivalent), the resulting mixture is stirred at 10-70°C (for example, 10-50°C, 10-40°C, 20-30°C, e.g. 25°C) for not less than 1 hour (for example 1-48 hours, 4-24 hours, 8-16 hours, e.g. not less than 10 hours), filtered, and the filter cake is washed with ethyl acetate, and dried to produce (3S,3aR)-2-(3-chloro-4-cyano-phenyl)-3-cyclopentyl-3,3a,4,5-tetrahydro-2H-pyrazolo[3,4-f]quino line-7-carboxylic acid quinine salt.

Technical Solution 4: A process for preparing (3S,3aR)-2-(3-chloro-4-cyano-phenyl)-3-cyclopentyl-3,3a,4,5-tetrahydro-2H-pyrazolo[3,4-f]quino line-7-carboxylic acid quinidine salt, which comprises the following steps:
At 10-40°C (for example, 20-30°C, e.g. 25°C), 2-(3-chloro-4-cyano-phenyl)-3 -cyclopentyl-3,3 a,4,5 -tetrahydro-2H-pyrazolo[3,4-f]quinoline-7-car boxylic acid (1 molar equivalent) and quinidine (not less than 0.2 molar equivalents, for example, 0.2-5 molar equivalents, 0.5-2 molar equivalents, e.g. 1 molar equivalent) are added to ethyl acetate (not less than 5.05 L/1 molar equivalent, for example 5.05-126.27 L/1 molar equivalent, for example 12.63-50.51 L/1 molar equivalent, e.g. 25253.4 mL/1 molar equivalent), the resulting mixture is stirred at 10-70°C (for example, 10-50°C, 10-40°C, 20-30°C, e.g. 25°C) for not less than 1 hour (for example 1-48 hours, 4-24 hours, 8-16 hours, e.g. not less than 10 hours), filtered, and the collected mother liquor is distilled to produce (3S,3aR)-2-(3-chloro-4-cyano-phenyl)-3-cyclopentyl-3,3a,4,5-tetrahydro-2H-pyrazolo[3,4-f]quino line-7-carboxylic acid quinidine salt.

Technical Solution 5: The process according to any of Technical Solutions 1-4, wherein 2-(3-chloro-4-cyano-phenyl)-3 -cyclopentyl-3,3 a,4,5 -tetrahydro-2H-pyrazolo[3,4-f]quinoline-7-car boxylic acid is obtained by the following steps:

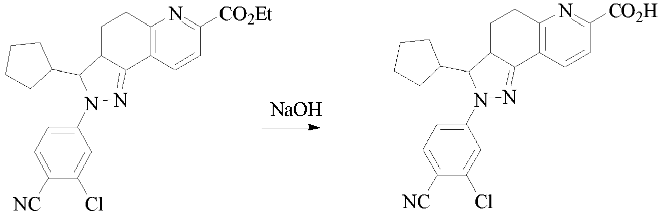

At 10-40°C (for example, 20-30°C, e.g. 25°C), 2-(3-chloro-4-cyano-phenyl)-3 -cyclopentyl-3,3 a,4,5 -tetrahydro-2H-pyrazolo[3,4-f]quinoline-7-car boxylic acid ethyl ester (1 molar equivalent), methanol (not less than 0.18 L/1 molar equivalent, for example, 0.18-4.49 L/1 molar equivalent, 0.45-1.80 L/1 molar equivalent, e.g. 898 mL/1 molar equivalent) and tetrahydrofuran (not less than 0.36 L/1 molar equivalent, for example, 0.36-8.98 L/1 molar equivalent, 0.90-3.59 L/1 molar equivalent, e.g. 1796 mL/1 molar equivalent) are mixed, the resulting mixture is cooled to 0-40°C (for example, 0-20°C, 0-10°C, e.g. 5°C);

To the resulting mixture is added dropwise 10% aqueous NaOH solution (not less than 0.40 molar equivalents, for example, 0.40-10.02 molar equivalents, 1.00-4.01 molar equivalents, e.g. 2 molar equivalents), and the mixture is stirred at 0-40°C (for example, 0-20°C, 0-10°C, e.g. 5°C) for not less than 0.1 hours (for example, 0.1-10 hours, 0.1-5 hours, 0.5-2 hours, e.g. 1 hour);

To the resulting mixture is added dropwise a diluted hydrochloric acid until the pH becomes 3-5, the resulting mixture is warmed up to 10-40°C (for example, 20-30°C, e.g. 25°C), stirred, filtered, and the filter cake is washed with methanol, and dried under vacuum to produce 2-(3-chloro-4-cyano-phenyl)-3 -cyclopentyl-3,3 a,4,5 -tetrahydro-2H-pyrazolo[3,4-f]quinoline-7-car boxylic acid.

Technical Solution 6: The process according to Technical Solution 5, wherein 2-(3-chloro-4-cyano-phenyl)-3 -cyclopentyl-3,3 a,4,5 -tetrahydro-2H-pyrazolo[3,4-f]quinoline-7-car boxylic acid ethyl ester is obtained by the following steps:

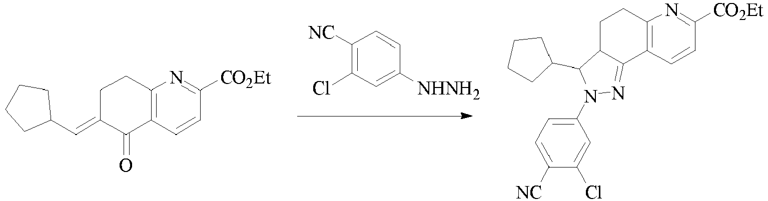

At 10-40°C (for example, 20-30°C, e.g. 25°C), 6-cyclopentylmethylene-5-oxo-5,6,7,8-tetrahydro-quinoline-2-carboxylic acid ethyl ester (1 molar equivalent), 2-chloro-4-hydrazino benzonitrile (not less than 0.21 molar equivalents, for example, 0.21-5.22 molar equivalents, 0.52-2.09 molar equivalents, e.g. 1.05 molar equivalents), ethanol (not less than 0.19 L/1 molar equivalent, for example, 0.19-4.79 L/1 molar equivalent, 0.48-1.92 L/1 molar equivalent, e.g. 958 mL/1 molar equivalent) and a solution of hydrogen chloride in ethanol (at a concentration of 2.0 mol/L, not less than 0.11 L/1 molar equivalent, for example, 0.11-2.75 L/1 molar equivalent, 0.28-1.10 L/1 molar equivalent, e.g. 551 mL/1 molar equivalent) are mixed, and the resulting mixture is warmed up to 40-90°C (for example, 70-80°C, e.g. 75°C), and reacted in darkness for not less than 3 hours (for example, 3-80 hours, 8-48 hours, 8-32 hours, e.g. 16 hours); the resulting mixture is cooled down to 0-20°C (for example, 0-10°C, e.g. 5°C), stirred, and filtered by suction; and the filter cake is washed with absolute ethanol, and dried under vacuum to produce 2-(3-chloro-4-cyano-phenyl)-3 -cyclopentyl-3,3 a,4,5 -tetrahydro-2H-pyrazolo[3,4-f]quinoline-7-car boxylic acid ethyl ester.

Technical Solution 7: The process according to Technical Solution 6, wherein 2-chloro-4-hydrazino benzonitrile is obtained by the following steps:

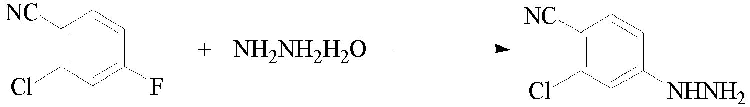

At 10-40°C (for example, 20-30°C, e.g. 25°C), 2-chloro-4-fluorobenzonitrile (1 molar equivalent), hydrazine hydrate (not less than 0.60 molar equivalents, for example, 0.60-15.04 molar equivalents, 1.5-6.01 molar equivalents, e.g. 3 molar equivalents) and absolute ethanol (not less than 156 g/1 molar equivalent, for example 156-3892 g/1 molar equivalent, 389-1557 g/1 molar equivalent, e.g. 778 g/1 molar equivalent) are mixed; the resulting mixture is warmed up to 40-90°C (for example, 75-85°C, e.g. 80°C) and reacted for not less than 0.5 hours (for example, 0.5-15 hours, 1-5 hours, e.g. 3 hours); to the resulting mixture is added water (not less than 311 g/1 molar equivalent, for example, 311-7783 g/1 molar equivalent, 778-3113 g/1 molar equivalent, e.g. 1557 g/1 molar equivalent); the resulting mixture is cooled down to 5-40°C (for example, 10-30°C, e.g. 20°C), stirred, and filtered by suction; and the filter cake is rinsed with ethanol, and the wet filter cake is dried under vacuum to produce 2-chloro-4-hydrazino benzonitrile.

Technical Solution 8: The process according to Technical Solution 6, wherein 6-cyclopentylmethylene-5-oxo-5,6,7,8-tetrahydro-quinoline-2-carboxylic acid ethyl ester is obtained by the following steps:

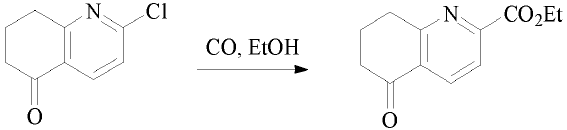

At 10-40°C (for example, 20-30°C, e.g. 25°C), to a high-pressure autoclave are added 2-chloro-7,8-dihydro-6H-quinolin-5-one (1 molar equivalent), absolute ethanol (not less than 0.18 L/1 molar equivalent, for example, 0.18-4.54 L/1 molar equivalent, 0.45-1.82 L/1 molar equivalent, e.g. 908 mL/1 molar equivalent), N,N-dimethylformamide (DMF) (not less than 18 mL/1 molar equivalent, for example 18-454 mL/1 molar equivalent, 45-182 mL/1 molar equivalent, e.g. 91 mL/1 molar equivalent), triethylamine (not less than 1 molar equivalents, for example 1-4 molar equivalents, 0.4-10 molar equivalents, e.g. 2 molar equivalents) and Pd(dppf)Ch₂•CH₂Cl₂ (not less than 3 g/1 molar equivalent, for example, 3-73 g/1 molar equivalent, 7-29 g/1 molar equivalent, e.g. 15 g/1 molar equivalent); the high-pressure autoclave is transformed to the CO gas, pressurized to 0.5-8MPa (for example 1-4 MPa, e.g. 2 MPa), warmed up to an internal temperature of 40-90°C (for example, 70-80°C, e.g. 75°C), and reacted for 0.5-15 hours (for example not less than 0.5 hours, 1-5 hours, e.g. 3 hours); the reaction system is cooled down to about 35-80°C (for example 50-70°C, e.g. 60°C) and distilled under vacuum until no fraction is produced, and the concentration is completed to produce a concentrated liquor; to the concentrated liquor is added ethyl acetate, and the resulting mixture is stirred at room temperature, and filtered by suction; the filter cake is rinsed with ethyl acetate, hydrochloric acid is added, and the mixture is stirred and allowed to stand by and separated into layers to obtain an aqueous phase and an organic phase; ethyl acetate is added to the aqueous phase, and the mixture is stirred and allowed to stand by and separated into layers; the organic phases are combined, and the combined organic phase is distilled under vacuum until no fraction is produced to produce an oily substance, which is directly used in the next reaction;

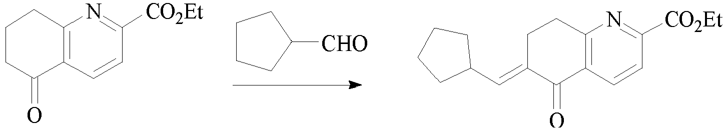

At 10-40°C (for example, 20-30°C, e.g. 25°C), the resulting oily substance and absolute ethanol (not less than 127 mL/1 molar equivalent, for example, 127-3178 mL/1 molar equivalent, 318-1271 mL/1 molar equivalent, e.g. 636 mL/1 molar equivalent) are mixed and the temperature is controlled at about 0°C±5°C (for example, 0°C±2°C, e.g. 0°C); to the resulting mixture is added cyclopentyl formaldehyde (not less than 0.2 molar equivalents, for example, 0.2-5 molar equivalents, 0.5-2 molar equivalents, e.g. 1 molar equivalent). The resulting mixture is stirred for 2-50 minutes (for example not less than 2 minutes, 5-20 minutes, e.g. 10 minutes), and the temperature is controlled at about 0°C±5°C (for example, 0°C±2°C, e.g. 0°C). To the resulting mixture is added dropwise pyrrolidine (not less than 0.12 molar equivalents, for example, 0.12-3 molar equivalents, 0.3-1.2 molar equivalents, e.g. 0.6 molar equivalents), and the mixture is warmed up to 0-40°C (for example, 10-30°C, e.g. 25°C); the resulting mixture is reacted in darkness for not less than 2 hours (for example, 2-40 hours, 4-24 hours, 4-16 hours, e.g. 8 hours). To the resulting mixture is added water; the resulting mixture is cooled down to 0-20°C (for example, 0-10°C, e.g. 5°C), stirred for not less than 0.1 hours (for example, 0.1-10 hours, 0.5-2 hours, e.g. 1 hour), and filtered by suction. The filter cake is washed with a mixed solvent of ethanol and water and dried under vacuum to produce 6-cyclopentylmethylene-5-oxo-5,6,7,8-tetrahydro-quinoline-2-carboxylic acid ethyl ester.

Technical Solution 9: The process according to Technical Solution 8, wherein 2-chloro-7,8-dihydro-6H-quinoline-5-one is obtained by the following steps:

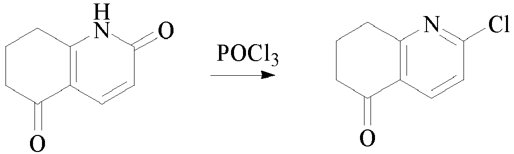

At room temperature, to a mixture of 7,8-dihydro-1H,6H-quinoline-2,5-dione (1 molar equivalent) and acetonitrile (not less than 261 g/1 molar equivalent, for example, 261-6527 g/1 molar equivalent, 653-2611 g/1 molar equivalent, e.g. 1305 g/1 molar equivalent) is added phosphorus oxychloride (not less than 0.34 molar equivalents, for example, 0.34-8.51 molar equivalents, 0.85-3.4 molar equivalents, e.g. 1.7 molar equivalents). The resulting mixture is warmed up to 40-90°C (for example, 75-85°C, e.g. 80°C) and reacted for not less than 0.5 hours (for example, 0.5-10 hours, 1-5 hours, e.g. 2 hours). Then the resulting mixture is cooled down to 30-65°C (for example 50-60°C, e.g. 55°C), and distilled under vacuum off 25-100wt% (for example 50-85wt%, e.g. 75wt%) of the solvent, and then the mixture is cooled down to 10-40°C (for example, 20-30°C, e.g. 25°C). Water is added to quench the reaction, the stirring is continued at 10-40°C (for example, 20-30°C, e.g. 25°C). The mixture is adjusted with an aqueous NaOH solution to a pH value of 5-7, and the resulting mixture is cooled down to 0-10°C, stirred, and filtered. The filter cake is rinsed with water and dried under vacuum to produce 2-chloro-7,8-dihydro-6H-quinoline-5-one.

Technical Solution 10: The process according to Technical Solution 1, wherein the prepared (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt is useful in preparation of (3S,3aR)-2-(3-chloro-4-cyano-phenyl)-3-cyclopentyl-3,3a,4,5-tetrahydro-2H-pyrazolo[3,4-f]quino line-7-carboxylic acid, which comprises the following steps:

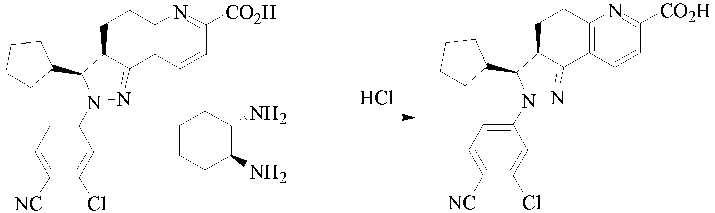

At 10-40°C (for example, 20-30°C, e.g. 25°C), (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt (1 molar equivalent) and ethanol (not less than 0.64 L/1 molar equivalent, for example, 0.64-16.05 L/1 molar equivalent, 1.61-6.42 L/1 molar equivalent, e.g. 3210 mL/1 molar equivalent) are added to 1 mol/L hydrochloric acid (not less than 0.21 molar equivalents, for example, 0.21-5.35 molar equivalents, 0.54-2.14 molar equivalents, e.g. 1.07 molar equivalents); the resulting mixture is stirred at 0-40°C (for example, 20-30°C, e.g. 25°C) for not less than 0.5 hours (for example, 0.5-10 hours, 1-5 hours, e.g. 2 hours), and filtered; and the filter cake is washed with ethanol, and dried under vacuum to produce (3S,3aR)-2-(3-chloro-4-cyano-phenyl)-3-cyclopentyl-3,3a,4,5-tetrahydro-2H-pyrazolo[3,4-f]quino line-7-carboxylic acid.

Technical Solution 11: The process according to Technical Solution 10, wherein the prepared (3S,3aR)-2-(3-chloro-4-cyano-phenyl)-3-cyclopentyl-3,3a,4,5-tetrahydro-2H-pyrazolo[3,4-f]quino line-7-carboxylic acid is useful in preparation of 2-chloro-4-[(3S,3aR)-3-cyclopentyl-7-(4-hydroxylpiperidine-1-carbonyl)-3,3a,4,5-tetrahydro-2H-pyrazolo[3,4-f]quinoline-2-yl]benzonitrile, which comprises the following steps:

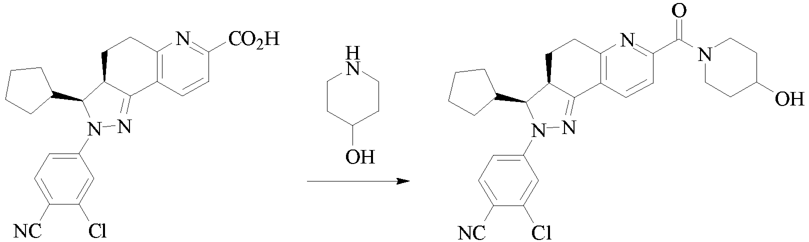

At 10-40°C (for example, 20-30°C, e.g. 25°C), (3S,3aR)-2-(3-chloro-4-cyano-phenyl)-3-cyclopentyl-3,3a,4,5-tetrahydro-2H-pyrazolo[3,4-f]quino line-7-carboxylic acid (1 molar equivalent), 2-(7-azabenzotriazol-1-yl)-N,N,N',N'-tetramethyluronium hexafluorophosphate (HATU) (not less than 0.24 molar equivalents, for example, 0.24-6 molar equivalents, 0.6-2.4 molar equivalents, e.g. 1.2 molar equivalents), methylene chloride (not less than 0.42 L/1 molar equivalent, for example, 0.42-10.52 L/1 molar equivalent, 1.05-4.21 L/1 molar equivalent, e.g. 2104 mL/1 molar equivalent), N-methylpyrrolidinone (NMP) (not less than 84 mL/1 molar equivalent, for example 84-2104 mL/1 molar equivalent, 210-842 mL/1 molar equivalent, e.g. 421 mL/1 molar equivalent) and triethylamine (not less than 0.3 molar equivalents, for example, 0.3-7.5 molar equivalents, 0.75-3 molar equivalents, e.g. 1.5 molar equivalents) are mixed; the resulting mixture is stirred at 0-40°C (for example, 20-30°C, e.g. 25°C) for not less than 3 minutes (for example, 3-300 minutes, 15-60 minutes, e.g. 30 minutes); then a solution of 4-hydroxylpiperidine (not less than 0.24 molar equivalents, for example, 0.24-6 molar equivalents, 0.6-2.4 molar equivalents, e.g. 1.2 molar equivalents) in methylene chloride (not less than 168 mL/1 molar equivalent, for example 168-4209 mL/1 molar equivalent, 421-1684 mL/1 molar equivalent, e.g. 842 mL/1 molar equivalent) is added dropwise to the above mixture; the resulting mixture is stirred at 0-40°C (for example, 20-30°C, e.g. 25°C) for not less than 0.2 hours (for example, 0.2-20 hours, 0.5-10 hours, 1-5 hours, e.g. 2 hours); to the resulting mixture is added an aqueous hydrochloric acid solution, and the mixture is stirred, allowed to stand by and separated into layers to produce an organic phase; to the resulting organic phase is added an aqueous sodium carbonate solution, and the mixture is stirred, allowed to stand by, and separated into layers to obtain an organic phase; to the resulting organic phase is added an aqueous solution, and the mixture is stirred, allowed to stand by, and separated into layers to obtain an organic phase; the organic phase is evaporated to dryness to produce an oily substance, the oily substance and isopropanol (not less than 1.01 kg/1 molar equivalent, for example 1.01-25.26 kg/1 molar equivalent, 2.53-10.10 kg/1 molar equivalent, e.g. 5.05 kg/1 molar equivalent) are mixed, and heated to 50-90°C (for example, 75-85°C, e.g. 80°C); the resulting solution is clear and cooled down to 10-40°C (for example, 20-30°C, e.g. 25°C), stirred for not less than 0.3 hours (for example, 0.3-30 hours, 1.5-6 hours, e.g. 3 hours), and filtered; and the filter cake is washed with isopropanol, and dried under vacuum to produce 2-chloro-4-[(3S,3aR)-3-cyclopentyl-7-(4-hydroxylpiperidine-1-carbonyl)-3,3a,4,5-tetrahydro-2H-pyrazolo [3,4-f]quinoline-2-yl]benzonitrile.

Technical Solution 12: The process according to Technical Solution 1, wherein the prepared (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt is useful in preparation of 2-chloro-4-[(3S,3aR)-3-cyclopentyl-7-(4-hydroxylpiperidine-1-carbonyl)-3,3a,4,5-tetrahydro-2H-pyrazolo[3,4-f]quinoline-2-yl]benzonitrile, which comprises the following steps:

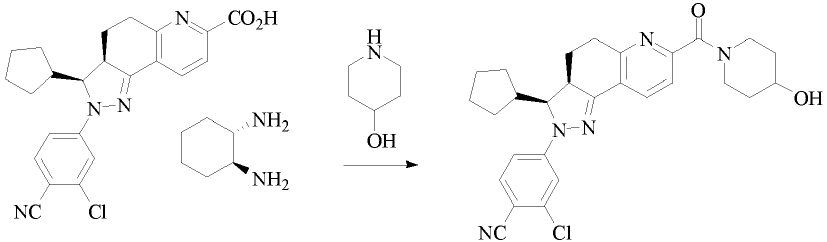

At 10-40°C (for example, 20-30°C, e.g. 25°C), (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt (1 molar equivalent), 2-(7-azabenzotriazol-1-yl)-N,N,N',N'-tetramethyluronium hexafluorophosphate (HATU) (not less than 0.24 molar equivalents, for example, 0.24-6 molar equivalents, 0.6-2.4 molar equivalents, e.g. 1.2 molar equivalents), methylene chloride (not less than 0.42 L/1 molar equivalent, for example, 0.42-10.52 L/1 molar equivalent, 1.05-4.21 L/1 molar equivalent, e.g. 2104 mL/1 molar equivalent), N-methylpyrrolidinone (NMP) (not less than 84 mL/1 molar equivalent, for example 84-2104 mL/1 molar equivalent, 210-842 mL/1 molar equivalent, e.g. 421 mL/1 molar equivalent) and triethylamine (not less than 0.3 molar equivalents, for example, 0.3-7.5 molar equivalents, 0.75-3 molar equivalents, e.g. 1.5 molar equivalents) are mixed; the resulting mixture is stirred at 10-40°C (for example, 20-30°C, e.g. 25°C) for not less than 3 minutes (for example, 3-300 minutes, 15-60 minutes, e.g. 30 minutes); then a solution of 4-hydroxylpiperidine (not less than 0.24 molar equivalents, for example, 0.24-6 molar equivalents, 0.6-2.4 molar equivalents, e.g. 1.2 molar equivalents) in methylene chloride (not less than 168 mL/1 molar equivalent, for example 168-4209 mL/1 molar equivalent, 421-1684 mL/1 molar equivalent, e.g. 842 mL/1 molar equivalent) is added dropwise to the above mixture; the resulting mixture is stirred at 10-40°C (for example, 20-30°C, e.g. 25°C) for not less than 0.2 hours (for example, 0.2-20 hours, 0.5-10 hours, 1-5 hours, e.g. 2 hours); to the resulting mixture is added an aqueous hydrochloric acid solution, and the mixture is stirred, allowed to stand by, and separated into layers to obtain an organic phase; to the resulting organic phase is added an aqueous sodium carbonate solution, and the mixture is stirred, allowed to stand by, and separated into layers to obtain an organic phase; to the resulting organic phase is added an aqueous solution, and the mixture is stirred, allowed to stand by, and separated into layers to obtain an organic phase; the organic phase is evaporated to dryness to produce an oily substance, the oily substance and isopropanol (not less than 1.01 kg/1 molar equivalent, for example 1.01-25.26 kg/1 molar equivalent, 2.53-10.10 kg/1 molar equivalent, e.g. 5.05 kg/1 molar equivalent) are mixed, and heated to 50-90°C (for example, 75-85°C, e.g. 80°C); the resulting solution is clear and cooled down to 10-40°C (for example, 20-30°C, e.g. 25°C), stirred for not less than 0.3 hours (for example, 0.3-30 hours, 1.5-6 hours, e.g. 3 hours), and filtered; the filter cake is washed with isopropanol, and dried under vacuum to produce 2-chloro-4-[(3S,3aR)-3-cyclopentyl-7-(4-hydroxylpiperidine-1-carbonyl)-3,3a,4,5-tetrahydro-2H-pyrazolo [3,4-f]quinoline-2-yl]benzonitrile.

For the process for preparing a (3S,3aR)-3-cyclopentyl-3,3a,4,5-tetrahydro-2H-pyrazolo[3,4-f]quinoline compound disclosed in the present invention, in the resolution step, the resolution method by using a chiral base replaces the resolution with the supercritical liquid chromatography, thereby realizing the large-scale industrial production and reducing the costs; the total yield of the process is increased by about 7 times; the post-treatment of the process is simplified, safe, and conducive to the quality control and the cost reduction.

### Specific embodiments

Hereinafter, the above-mentioned content of the present invention will be further described in detail through specific embodiments in the form of examples. However, it should not be understood that the scope of the above-mentioned subject of the present invention is limited to the following embodiments.

### Example 1: Preparation of 2-chloro-7,8-dihydro-6H-quinolin-5-one

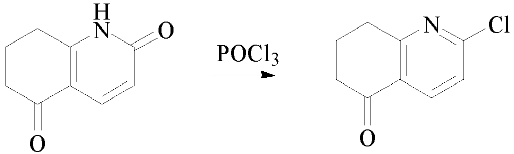

At room temperature, 50 g of 7,8-dihydro-1H,6H-quinoline-2,5-dione (1.0 eq), and 400 g of acetonitrile were added to a reaction vessel. 79.8 g of phosphorus oxychloride (1.7 eq) was added. The reaction vessel was warmed up to an internal temperature of 80°C. The reaction was performed for 2 hours. The reaction vessel was cooled down to 55°C. 300 g of solvent was removed by distillation under vacuum. After the completion of distillation, the reaction mixture was cooled down to 25°C, and water was added to quench the reaction mixture. After the completion of adding water, the mixture was further stirred at 25°C and adjusted with an aqueous NaOH solution at 25°C to a pH of 5-7. The resulting mixture was cooled down, stirred, and filtered. The filter cake was rinsed with water and dried under vacuum to produce 2-chloro-7,8-dihydro-6H-quinoline-5-one (49 g) as an off-white white in a yield of about 88%.

### Mass spectrum (M+1): 182

¹H-NMR (DMSO-d₆, 400MHz): δ 8.17 (1H, d), 7.51 (1H, d), 3.05 (2H, t), 2.65 (2H, t), 2.07-2.13 (2H, m).

### Example 2: Preparation of 5-oxo-5,6,7,8-tetrahydro-quinoline-2-carboxylic acid ethyl ester

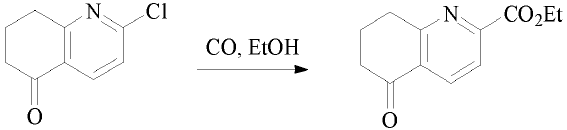

At 25°C, to a high-pressure autoclave were added 40 g of 2-chloro-7,8-dihydro-6H-quinoline-5-one (1.0 eq), 200 ml of absolute ethanol, 20 ml of DMF, 44.5 g of triethylamine (2.0 eq) and 3.2 g of Pd(dppf)Cl₂•CH₂Cl₂. The high-pressure autoclave was transformed to the CO gas, pressurized to 2 MPa, and warmed up to an internal temperature of 75°C. The reaction was carried out for 3 hours until the reaction was completed. The system was cooled down to about 60°C, and distilled under vacuum until no fraction was produced. After the completion of the concentration, ethyl acetate was added. The mixture was stirred at room temperature, and filtered by suction. The filter cake was rinsed with ethyl acetate. The mother liquors were combined, and hydrochloric acid was added. The mixture was stirred, allowed to stand by, and separated into layers to obtain an aqueous phase and an organic phase. Ethyl acetate was added to the aqueous phase, and the mixture was stirred and allowed to stand by and separated into layers. The organic phases were combined, and the combined organic phase was distilled under vacuum until no fraction was produced to produce a black oily substance (about 50 g), which was directly used in the next step reaction.

### Mass spectrum (M+1): 220

¹H-NMR (DMSO-d₆, 400MHz): δ 8.32 (1H, d), 8.00 (1H, d), 4.35-4.40 (2H, q), 3.14 (2H, t), 2.71 (2H, t), 2.10-2.17 (2H, m), 1.34 (3H, t).

### Example 3: Preparation of 6-cyclopentylmethylene-5-oxo-5,6,7,8-tetrahydro-quinoline-2-carboxylic acid ethyl ester

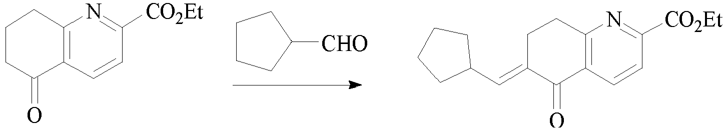

At 25°C, to a reaction vessel were added 50 g of the crude 5-oxo-5,6,7,8-tetrahydro-quinoline-2-carboxylic acid ethyl ester and 140 ml of absolute ethanol. The temperature was controlled at about 0°C. To the reaction vessel was added 21.7 g of cyclopentyl formaldehyde (1.0 eq). After the completion of the addition, the mixture was stirred for 10 minutes. The temperature was controlled at about 0°C. To the reaction vessel was added dropwise 9.4 g of pyrrolidine (0.6 eq). The system was warmed up to 25°C and reacted in darkness for 8 hours. Water was added. The reaction mixture was cooled down to 5±5°C, stirred for 1 hour, and filtered by suction. The filter cake was washed with a mixed solvent of ethanol and water and dried under vacuum to produce 6-cyclopentylmethylene-5-oxo-5,6,7,8-tetrahydro-quinoline-2-carboxylic acid ethyl ester (47.6 g) as a pale-yellow solid in a two-step yield of about 72%.

### Mass spectrum (M+1): 300

¹H-NMR (DMSO-d₆, 400MHz): δ 8.38 (1H, d), 8.03 (1H, d), 6.78 (1H, d), 4.35-4.40 (2H, q), 3.13 (2H, t), 2.87-2.93 (3H, m), 1.61-1.88 (6H, m), 1.34-1.40 (5H, t).

### Example 4: Preparation of 2-chloro-4-hydrazino benzonitrile

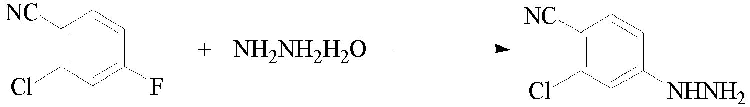

At 25°C, to a reaction vessel were added 40 g of 2-chloro-4-fluorobenzonitrile (1.0 eq), 45.5 g of hydrazine hydrate (3.0 eq, 85%) and 200 g of absolute ethanol. The mixture was warmed up to 80°C and reacted for 3 hours. 400 g of water was added. The mixture was cooled down to 20°C, stirred, and filtered by suction. The filter cake was rinsed with ethanol, and the wet filter cake was dried under vacuum to produce 2-chloro-4-hydrazino benzonitrile (about 40 g) as a white solid in a yield of about 92%.

### Mass spectrum (M+1): 168

¹H-NMR (DMSO-d₆, 400MHz): δ 8.09 (1H, s), 7.49 (1H, d), 6.91 (1H, s), 6.69 (1H, d), 4.40 (2H,s).

### Example 5: Preparation of 2-(3-chloro-4-cyano-phenyl)-3 -cyclopentyl-3,3 a,4,5 -tetrahydro-2H-pyrazolo[3,4-f]quinoline-7-car boxylic acid ethyl ester

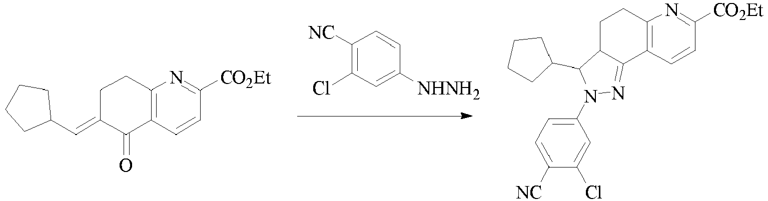

At 25°C, to a reaction vessel were added 20 g of 6-cyclopentylmethylene-5-oxo-5,6,7,8-tetrahydro-quinoline-2-carboxylic acid ethyl ester (1.0 eq), 11.7 g of 2-chloro-4-hydrazino benzonitrile (1.05 eq), 64 ml of ethanol, and 36.8 ml of a solution of hydrogen chloride in ethanol (2.0 mol/L). The mixture was warmed up to an internal temperature of 75°C, reacted in darkness for 16 hours, then cooled down to 5±5°C, stirred, and filtered by suction. The filter cake was washed with absolute ethanol, and dried under vacuum to produce 2-(3-chloro-4-cyano-phenyl)-3 -cyclopentyl-3,3 a,4,5 -tetrahydro-2H-pyrazolo[3,4-f]quinoline-7-car boxylic acid ethyl ester (22.5 g) as a yellow solid in a yield of about 75%. Mass spectrum (M+1): 449 ¹H-NMR (DMSO-d₆, 400MHz): δ 8.47 (1H, d), 7.95 (1H, d), 7.71 (1H, d), 7.45 (1H, s), 7.27 (1H, d), 5.01-5.05 (1H, m), 4.34-4.39 (2H, q), 3.67-3.74 (1H, m), 3.02-3.19 (2H, m), 1.40-2.30 (14H, m).

### Example 6: Preparation of 2-(3-chloro-4-cyano-phenyl)-3 -cyclopentyl-3,3 a,4,5 -tetrahydro-2H-pyrazolo[3,4-f]quinoline-7-car boxylic acid

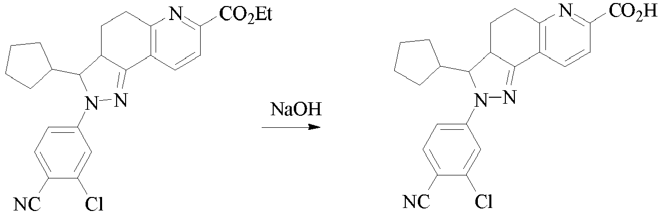

At 25°C, to a reaction vessel were added 7 g of 2-(3-chloro-4-cyano-phenyl)-3 -cyclopentyl-3,3 a,4,5 -tetrahydro-2H-pyrazolo[3,4-f]quinoline-7-car boxylic acid ethyl ester (1.0 eq), 14 ml of methanol and 28 ml of tetrahydrofuran. The reaction mixture was cooled down to 5±5°C. 12.5 g of 10% aqueous NaOH solution (2.0 eq) was added dropwise. The mixture was stirred at 5±5°C for 1 hour. A diluted hydrochloric acid was added dropwise to a pH value of 3-5. The resulting mixture was warmed up to 25±5°C, stirred, and filtered. The filter cake was washed with methanol, and dried under vacuum to produce 2-(3-chloro-4-cyano-phenyl)-3 -cyclopentyl-3,3 a,4,5 -tetrahydro-2H-pyrazolo[3,4-f]quinoline-7-car boxylic acid (6.23 g) as a yellow solid in a yield of about 95%.

### Mass spectrum (M+1): 421

¹H-NMR (CDCl₃-d₃, 400MHz): δ 8.54 (1H, d), 8.12 (1H, d), 7.47 (1H, d), 7.33 (1H, s), 7.03 (1H, d), 4.72-4.76 (1H, m), 3.05-3.62 (3H, m), 1.23-2.43 (11H, m).

### Example 7: Preparation of (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt

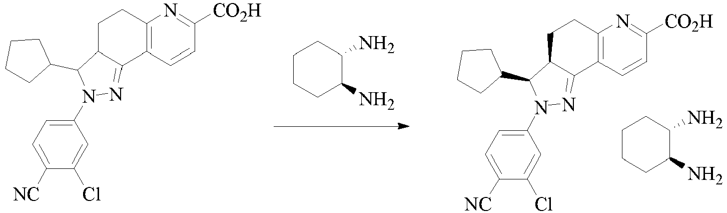

At 25°C, to a reaction vessel were added 5.0 g of 2-(3-chloro-4-cyano-phenyl)-3 -cyclopentyl-3,3 a,4,5 -tetrahydro-2H-pyrazolo[3,4-f]quinoline-7-car boxylic acid (1.0 eq) and 2.71 g of (1S,2S)-cyclohexane diamine (2.0 eq). 500 ml of acetone was added. The reaction mixture was stirred at 25°C for not less than 10 hours and filtered. The filter cake was washed with acetone. A filter cake was sampled and subjected to the HPLC test, showing the PSC/1 chiral purity of not less than 95%. The filter cake was dried under vacuum to produce (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt (2.3 g) as a yellow solid in a yield of about 35%. The product was taken as a liquid phase and was consistent with the control.

### Mass spectrum (M+1): 421

¹H-NMR (CDCl₃-d₃, 400MHz): δ 8.35 (1H, d), 8.04 (1H, d), 7.43 (1H, d), 7.27 (1H, s), 6.96 (1H, d), 4.60-4.64 (1H, m), 2.90-3.84 (5H, m), 1.01-2.41 (23H, m).

### Example 8: Preparation of (3S,3aR)-2-(3-chloro-4-cyano-phenyl)-3-cyclopentyl-3,3a,4,5-tetrahydro-2H-pyrazolo[3,4-f]quino line-7-carboxylic acid (1R,2R)-cyclohexane diamine salt

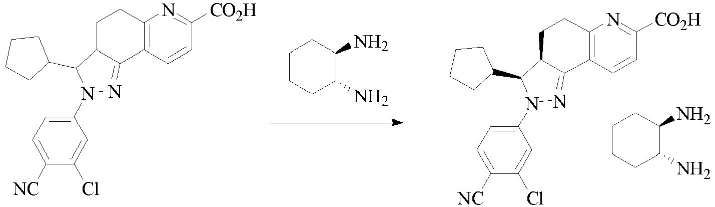

According to the process of Example 7, the reaction mixture was filtered, and the collected mother liquor was distilled to produce (3S,3aR)-2-(3-chloro-4-cyano-phenyl)-3-cyclopentyl-3,3a,4,5-tetrahydro-2H-pyrazolo[3,4-f]quino line-7-carboxylic acid (1R,2R)-cyclohexane diamine salt, which was subjected to the HPLC test, showing the PSC/1 chiral purity of not less than 95%.

### Example 9: Preparation of (3S,3aR)-2-(3-chloro-4-cyano-phenyl)-3-cyclopentyl-3,3a,4,5-tetrahydro-2H-pyrazolo[3,4-f]quino line-7-carboxylic acid quinine salt

At 25°C, to a reaction vessel were added 5.0 g 2-(3-chloro-4-cyano-phenyl)-3 -cyclopentyl-3,3 a,4,5 -tetrahydro-2H-pyrazolo[3,4-f]quinoline-7-car boxylic acid (1 eq) and 3.8 g of quinine (1 eq). Ethyl acetate (300 mL) was added. The reaction mixture was stirred at 25°C for not less than 10 hours and filtered. The filter cake was washed with ethyl acetate and dried to produce (3S,3aR)-2-(3-chloro-4-cyano-phenyl)-3-cyclopentyl-3,3a,4,5-tetrahydro-2H-pyrazolo[3,4-f]quino line-7-carboxylic acid quinine salt, which was subjected to the HPLC test, showing the PSC/1 chiral purity of not less than 75%.

### Example 10: Preparation of (3S,3aR)-2-(3-chloro-4-cyano-phenyl)-3-cyclopentyl-3,3a,4,5-tetrahydro-2H-pyrazolo[3,4-f]quino line-7-carboxylic acid quinidine salt

According to the process of Example 9, the reaction mixture was filtered, and the collected mother liquor was distilled to produce (3S,3aR)-2-(3-chloro-4-cyano-phenyl)-3-cyclopentyl-3,3a,4,5-tetrahydro-2H-pyrazolo[3,4-f]quino line-7-carboxylic acid quinidine salt, which was subjected to the HPLC test, showing the PSC/1 chiral purity of not less than 75%.

### Example 11: Preparation of (3S,3aR)-2-(3-chloro-4-cyano-phenyl)-3-cyclopentyl-3,3a,4,5-tetrahydro-2H-pyrazolo[3,4-f]quino line-7-carboxylic acid

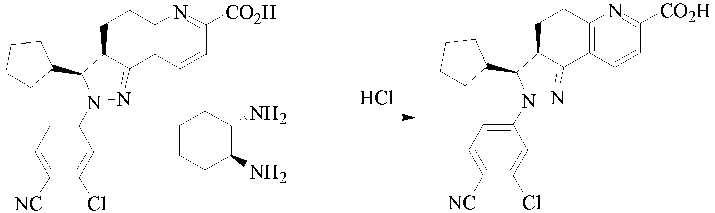

At 25°C, to a reaction vessel were added 5 g of (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt (1.0 eq) and 30 ml of ethanol. 10 ml of hydrochloric acid (1 mol/L, 1.07 eq) was added. The reaction mixture was stirred at 25°C for 2 hours and filtered. The filter cake was washed with ethanol. A filter cake was sampled. The filter cake was dried under vacuum to produce (3S,3aR)-2-(3-chloro-4-cyano-phenyl)-3-cyclopentyl-3,3a,4,5-tetrahydro-2H-pyrazolo[3,4-f]quino line-7-carboxylic acid (3.7 g) as a yellow solid in a yield of 94%.

### Example 12: Preparation of 2-chloro-4-[(3S,3aR)-3-cyclopentyl-7-(4-hydroxylpiperidine-1-carbonyl)-3,3a,4,5-tetrahydro-2H-pyrazolo [3,4-f]quinoline-2-yl]benzonitrile

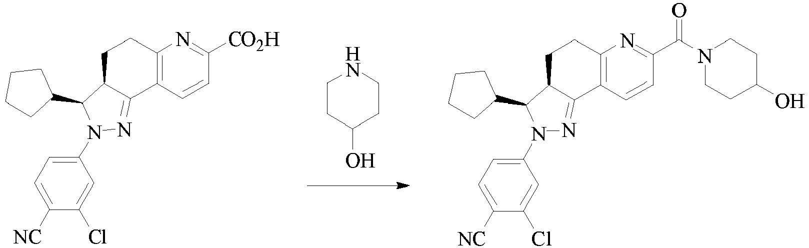

At 25°C, to a reaction vessel were added 10 g of (3S,3aR)-2-(3-chloro-4-cyano-phenyl)-3-cyclopentyl-3,3a,4,5-tetrahydro-2H-pyrazolo[3,4-f]quino line-7-carboxylic acid (1.0 eq), 10.8 g of 2-(7-azabenzotriazol-1-yl)-N,N,N',N'-tetramethyluronium hexafluorophosphate (HATU) (1.2 eq), 50 ml of methylene chloride and 10 ml of NMP. 3.6 g of triethylamine (1.5 eq) was added. The reaction mixture was stirred at 25°C for 30 minutes. 2.9 g of 4-hydroxylpiperidine (1.2 eq) was firstly dissolved in 20 ml of methylene chloride and then added dropwise to the reaction vessel. The reaction mixture was stirred at 25°C for 2 hours and sampled. An aqueous hydrochloric acid solution was added. The mixture was stirred, allowed to stand by, and separated into layers. An aqueous sodium carbonate solution was added. The mixture was stirred, allowed to stand by, and separated into layers. An aqueous solution was added. The mixture was stirred, allowed to stand by, and separated into layers. The organic phase was evaporated to dryness to produce 18 g of a yellow-brown oily substance. The oily substance and 120 g of an isopropanol solution were added to the reaction vessel and heated to 80°C. The resulting solution was clear and cooled down to 25°C, and then the mixture was stirred for more than 3 hours and filtered. The filter cake was washed with isopropanol, and dried under vacuum to produce 2-chloro-4-[(3S,3aR)-3-cyclopentyl-7-(4-hydroxylpiperidine-1-carbonyl)-3,3a,4,5-tetrahydro-2H-pyrazolo[3,4-f]quinoline-2-yl]benzonitrile (8.4 g) as a yellow solid in a yield of about 70%. The product was taken as a liquid phase and was consistent with the control.

### Example 13: Preparation of 2-chloro-4-[(3S,3aR)-3-cyclopentyl-7-(4-hydroxylpiperidine-1-carbonyl)-3,3a,4,5-tetrahydro-2H-pyrazolo [3,4-f]quinoline-2-yl]benzonitrile

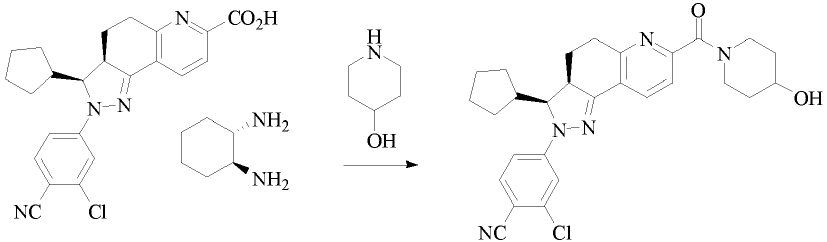

According to the process of Example 12, (3S,3aR)-2-(3-chloro-4-cyano-phenyl)-3-cyclopentyl-3,3a,4,5-tetrahydro-2H-pyrazolo[3,4-f]quino line-7-carboxylic acid (1S,2S)-cyclohexane diamine salt and 4-hydroxylpiperidine were reacted to produce 2-chloro-4-[(3S,3aR)-3-cyclopentyl-7-(4-hydroxylpiperidine-1-carbonyl)-3,3a,4,5-tetrahydro-2H-pyrazolo [3,4-f]quinoline-2-yl]benzonitrile.

## Claims

1. A process for preparing a compound of formula (III), wherein the compound of formula (III) is obtained through the following step (g):
(g) reacting a compound of formula (IV) and a chiral base Y to produce the compound of formula (III), 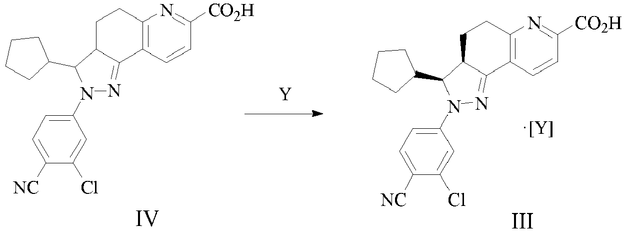 said chiral base Y is selected from (1S,2S)-cyclohexane diamine, (1R,2R)-cyclohexane diamine, quinine, quinidine, or deuterated derivatives thereof.

2. The process of claim 1, wherein said step (g) is a reaction in a ketone solvent, an ester solvent, or tetrahydrofuran.

3. The process of claim 1 or 2, wherein the compound of formula (IV) is obtained by the following step (f):
(f) A compound of formula (V) is hydrolyzed to produce the compound of formula (IV) 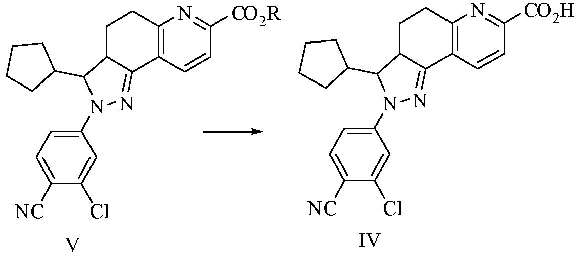 wherein R is C₁₋₆alkyl.

4. The process of claim 3, wherein said compound of formula (V) is obtained by the following step (e):
(e) A compound of formula (VII) and a compound of formula (VI) are reacted to produce the compound of formula (V) 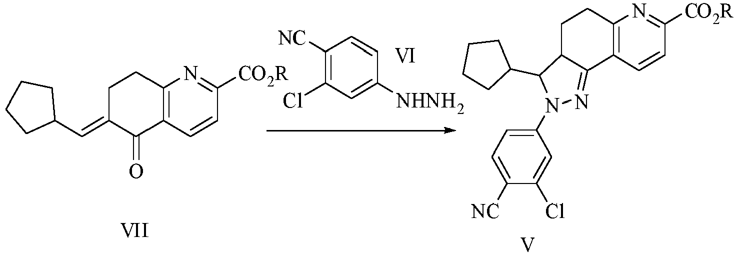 wherein R is C₁₋₆alkyl.

5. The process of claim 4, wherein said compound of formula (VI) is obtained by the following step (d):
(d) 2-chloro-4-fluoro benzonitrile and hydrazine hydrate are reacted to produce the compound of formula (VI)

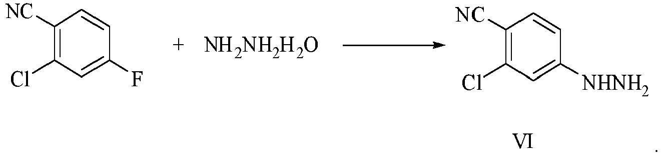

6. The process of claim 4, wherein said compound of formula (VII) is obtained by the following step (c):
(c) a compound of formula (VIII) and cyclopentyl formaldehyde are reacted to produce the compound of formula (VII) 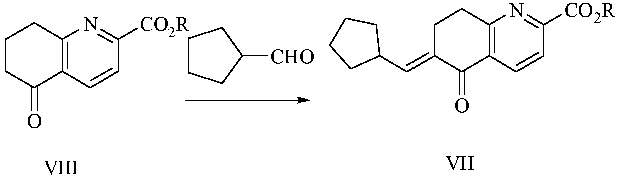 wherein R is C₁₋₆alkyl.

7. The process of claim 6, wherein said compound of formula (VIII) is obtained by the following step (b):
(b) The compound of formula (VIII) is prepared from a compound of formula (IX) and ROH, preferably the compound of formula (VIII) is prepared from the compound of formula (IX) and ROH in the presence of CO 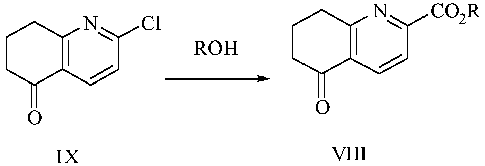 wherein R is C₁₋₆alkyl.

8. The process of claim 7, wherein said compound of formula (IX) is obtained by the following step (a):
(a) The compound of formula (IX) is prepared from a compound of formula (X), preferably the compound of formula (IX) is prepared from the compound of formula (X) and an acyl chloride compound, 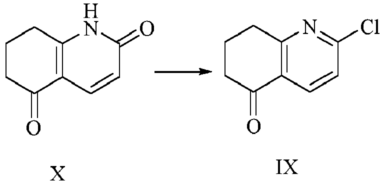 wherein the acyl chloride compound is selected from phosphoryl chloride, carbonyl chloride, and sulfuric chloride.

9. The process of claim 6, wherein said compound of formula (VIII) is obtained by the following steps (a) and (b):
(a) The compound of formula (IX) is prepared from the compound of formula (X), preferably the compound of formula (IX) is prepared from the compound of formula (X) and an acyl chloride compound, wherein the acyl chloride compound is selected from phosphoryl chloride, carbonyl chloride, and sulfuric chloride;
(b) The compound of formula (VIII) is prepared from the compound of formula (IX) and ROH, preferably the compound of formula (VIII) is prepared from the compound of formula (IX) and ROH in the presence of CO, 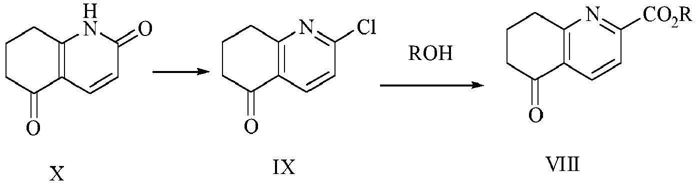
wherein R is C₁₋₆alkyl.

10. The process according to any of claims 1-8, wherein the process also comprises using the compound of formula (III) to prepare a compound of formula (II), which comprises the following steps:
(h) The compound of formula (III) is added to an acid solution and reacted to produce the compound of formula (II), 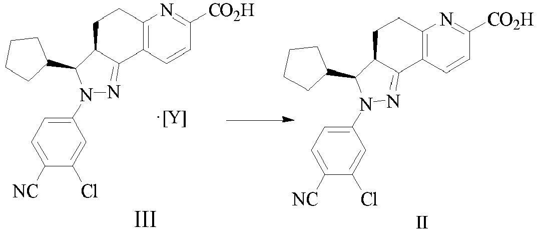 wherein Y is selected from (1S,2S)-cyclohexane diamine, (1R,2R)-cyclohexane diamine, quinine, quinidine, or deuterated derivatives thereof.

11. The process according to any of claims 1-8, wherein the process also comprises using the compound of formula (III) to prepare a compound of formula (I), which comprises the following steps: 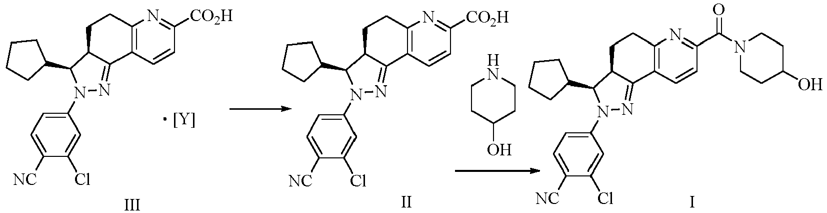
(h) The compound of formula (III) is added to an acid solution and reacted to produce a compound of formula (II),
(i) the compound of formula (II) and 4-hydroxylpiperidine are reacted to produce the compound of formula (I), wherein Y is selected from (1S,2S)-cyclohexane diamine, (1R,2R)-cyclohexane diamine, quinine, quinidine or deuterated derivatives thereof.

12. The process according to any of claims 1-8, wherein the process also comprises using the compound of formula (III) to prepare a compound of formula (I), which comprises the following steps:
The compound of formula (III) and 4-hydroxylpiperidine are reacted to produce the compound of formula (I), 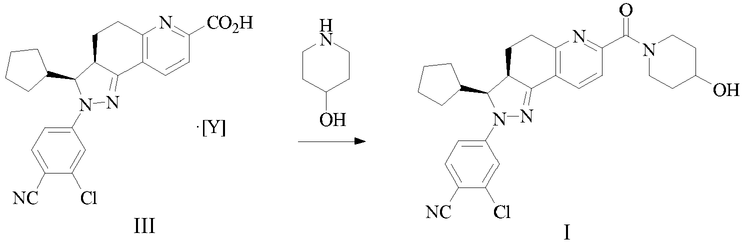 wherein Y is selected from (1S,2S)-cyclohexane diamine, (1R,2R)-cyclohexane diamine, quinine, quinidine, or deuterated derivatives thereof.

13. A compound of formula (III), which has the following structure: 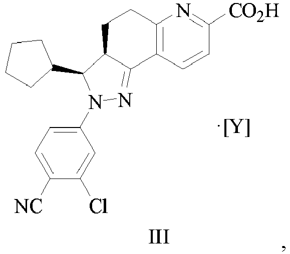 wherein Y is selected from (1S,2S)-cyclohexane diamine, (1R,2R)-cyclohexane diamine, quinine, quinidine, or deuterated derivatives thereof.
